(19) Europäisches Patentamt
European Patent Office
Office européen des brevets

(11) Publication number: **0 079 125**
**B1**

(12) # EUROPEAN PATENT SPECIFICATION

(45) Date of publication of patent specification: **05.04.89**

(21) Application number: **82305299.8**

(22) Date of filing: **05.10.82**

(51) Int. Cl.⁴: **C 07 D 493/04,** C 07 H 15/22, C 07 D 309/32 // (C07D493/04, 319:00, 311:00)

(54) **3-Substituted spectinomycin analogues and their preparation.**

(30) Priority: **23.10.81 US 314261**

(43) Date of publication of application: **18.05.83 Bulletin 83/20**

(45) Publication of the grant of the patent: **05.04.89 Bulletin 89/14**

(84) Designated Contracting States: **BE CH DE FR GB IT LI NL SE**

(56) References cited:
**DE-A-2 756 912**
**DE-A-2 851 953**
**US-A-4 351 771**
**THE JOURNAL OF ANTIBIOTICS, vol. 34, no. 1, January 1981, E. WOITUN et al. "Modification of spectinomycin - 2. Derivatives of 4-dihydro-4-deoxy-4(R)-aminospectinomycin", pages 22-27**

(73) Proprietor: **THE UPJOHN COMPANY**
**301 Henrietta Street**
**Kalamazoo, Michigan 49001 (US)**

(72) Inventor: **Thomas, Richard Charles**
**c/o The Upjohn Company 301 Henrietta Street**
**Kalamazoo Michigan 49001 (US)**
Inventor: **Fritzen, Edward Lawrence**
**c/o The Upjohn Company 301 Henrietta Street**
**Kalamazoo Michigan 49001 (US)**

(74) Representative: **Perry, Robert Edward et al**
**GILL JENNINGS & EVERY 53-64 Chancery Lane**
**London WC2A 1HN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 079 125 B1

## Description

The present invention relates to 3-substituted analogues of the aminocyclitol antibiotic spectinomycin.

Spectinomycin is a natural product, and is disclosed in US—A—3234902. Knight and Hoeksema, J. Antibiotics *28*, 136 (1975), disclose the N,N'-di(carbobenzyloxy) derivative of spectinomycin itself and both epimers of the 3'-dihydro analogue. The nitrogen atoms bonded to C—1 and C—3 are designated herein as N and N', respectively.

US—A—4173647, DE—A—2756912 and DE—A—2756913 disclose N,N'-blocked derivatives of spectinomycin, and also the reduction of spectinomycin hydrazones and oximes to both epimers of 3'-deoxy-3'-amino-3'-dihydrospectinomycin, advantageously in the presence of acid. C—6' analogues, including the 5'-desmethyl derivative, of spectinomycin, and their N-blocked derivatives, are disclosed in US—A—4351771.

Woitun *et al*, J. Antibiotics *34* (1981) 22—27, disclose 3'-substituted amino analogues of 3'-deoxo-3'-(R)-3'-amino-3'-dihydrospectinomycin and its N,N'-bicarbobenzyloxy derivative, and that certain analogues are inactive as anti-microbial agents. Analogues of both 3'-epimers of 3'-dihydrospectinomycin are reported in GB—A—2048243.

A review of hydrocyanation reactions of aldehydes and ketones, and their stereoselectivity, is provided by Mathieu *det al., Formation of C—C Bonds*, G. Thieme Publ. Stuttgart, W. Germany, Vol. I, pp. 429—440 (1973). See also Grewe *et al.*, Chem. Ber. *98*, 104 (1965).

Novel compounds, according to a first aspect of the invention, are of formula I (see below) wherein R is H, $C_{1-8}$ alkyl, $C_{1-4}$ hydroxyalkyl, ($C_{1-6}$alkoxy)$C_{1-4}$ alkyl having up to 7C atoms in total, $C_{1-4}$ aminoalkyl, ($C_{1-6}$alkylamino)$C_{1-4}$ alkyl having up to 7 C atoms in total, [di($C_{1-6}$ alkyl)amino]$C_{1-4}$ alkyl in which the $C_{1-4}$ alkyl group and either $C_{1-6}$ alkyl group together have up to 7C atoms, $C_{1-4}$ haloalkyl, $C_{1-4}$ dihaloalkyl or $C_{1-4}$ trihaloalkyl; and

one of $A_1$ and $A_2$ is OH and the other if CN, $CH_2CN$ or $CH_2NR_1R_2$ in which either $NR_1R_2$ is a heterocyclic radical having 3 to 10 ring atoms or $R_1$ and $R_2$ are independently selected from H, $C_{1-12}$ alkyl, $C_{6-12}$ aryl, optionally mono- or di- ring-substituted $C_{7-12}$ aralkyl, $C_{3-10}$ cycloalkyl, ($C_{3-10}$ cycloalkyl)$C_{1-4}$ alkyl and acyl selected from ($C_{1-12}$ alkyl)carbonyl, (optionally mono- or di-ring-substituted $C_{7-12}$ aralkyl)carbonyl, ($C_{3-10}$ cycloalkyl)carbonyl, [($C_{3-10}$ cycloalkyl)$C_{1-4}$ alkyl]carbonyl and optionally ring-substituted pyridylcarbonyl, piperazylcarbonyl, pyrrolylcarbonyl or morpholinocarbonyl, and any ring substituents are independently selected from F, Cl, I, $NH_2$, $C_{1-6}$ alkylamino, di($C_{1-6}$ alkyl)amino, CN, OH, COOH, ($C_{1-4}$ alkyl)carbonyl and $C_{1-4}$ alkoxy;

including pharmacologically-acceptable acid addition salts thereof.

Novel compounds, according to a second aspect of the invention, are of formula $II_b$ wherein $R_7$ is a blocking group;

R' is H, $C_{1-8}$ alkyl, $C_{1-4}$ hydroxyalkyl, ($C_{1-6}$ alkoxy)$C_{1-4}$ alkyl having up to 7 C atoms in total, N-blocked $C_{1-4}$ aminoalkyl, N-blocked ($C_{1-6}$ alkylamino)$C_{1-4}$ alkyl, [di($C_{1-4}$ alkyl)amino]$C_{1-4}$ alkyl in which the $C_{1-4}$ alkyl group and either $C_{1-6}$ alkyl group together have up to 7 C atoms, $C_{1-4}$ haloalkyl, $C_{1-4}$ dihaloalkyl or $C_{1-4}$ trihaloalkyl; and

either $A_3$ and $A_4$ are as defined for $A_1$ and $A_2$ above, or one of $A_3$ and $A_4$ is OH and the other is $CH_2N_3$.

A process, according to a third aspect of the invention, for preparing a mixture of epimers of a compound of formula I when $A_1$ or $A_2$ is CN or a mixture of epimers of a compound of formula $II_b$ when $A_3$ or $A_4$ is CN, comprises subjecting to hydrocyanation a compound of formula $III_{(b)}$ wherein $R_8$ is H and $R_3$ is R as defined above or $R_8$ is a blocking group and $R_3$ is R' as defined above, in the presence of a base or a basic ion-exchange resin, in an inert organic solvent; and either allowing the reaction product to remain in solution for a time sufficient that, predominantly in the mixture, $A_1$ or $A_3$ is CN; or terminating the reaction when, predominantly in the mixture, $A_2$ or $A_4$ is CN, either by the addition of acid to neutralise the base or basic ion-exchange resin or, if no other base is present, removing the resin.

Surprisingly, with respect to Mathieu *et al* and Grewe *et al, supra*, the 3-(R)-spectinomycin cyanohydrin is thermodynamically less stable than the 3-(S)-epimer. The latter ($A_1/A_3$ = CN) is therefore predominant in the mixture if the reaction product is allowed to remain in solution. The former ($A_2/A_4$ = CN) can be predominant if the reaction is terminated.

Compounds of formula $II_b$ can be used as intermediates in the preparation of compounds of formula I. Compounds of formula I are useful as antimicrobial agents which are microbistatic or microbicidal. They can be used to inhibit the growth of, or eliminate, microorganisms, including gram-negative and gram-positive bacteria, from environments in the presence of microorganisms is undesirable or harmful, and some are useful for treating or preventing microbial, especially bacterial, infections in mammals, including humans.

To be effective as microbistatic or microbicidal agents in an environment, the antimicrobial compounds within the scope of this invention must be introduced into the environment, by one of several means which are well known in the art and which are described in more detail below, in a quantity sufficient to inhibit the growth of, or eliminate, the target micro-organisms.

The anti-microbial activity of compounds within the scope of the invention has been determined by a serial-dilution technique. For each of several species of micro-organisms, the MIC, i.e. minimum concentration of a compound required to prevent an increase in the concentration of cells in an inoculum

containing a known initial concentration of cells in a growth phase was determined.

MIC values (in µg/ml) were obtained for spectinomycin dihydrochloride pentahydrate (Compound A, control) and three compounds (B, C and D) of the invention, and are reported in the following Table. Ranges reflect values obtained on different days.

| Organism | UC code No. | Compound A | B | C | D |
|---|---|---|---|---|---|
| *S. aureus* | 76 | 7.8 | 3.9 | 3.9 | ≦0.25 |
| *S. faecalis* | 694 | 31.2—62.5 | 125 | 125 | 3.9 |
| *E. coli* | 45 | 3.9—7.8 | 1.0 | 0.5 | 1.0 |
| *K. pneumoniae* | 58 | 2.0 | 1.0 | 1.0 | 3.9 |
| *Ps. aeruginosa* | 95 | 31.2 | 31.2 | 62.5 | 7.8 |
| *P. vulgaris* | 93 | 7.8 | 3.9 | 15.6 | 31.2 |
| *P. mirabilis* | 667 | 3.9—7.8 | 7.8 | 7.8 | 62.5 |
| *S. flexneri* | 143 | 7.8—15.6 | 3.9 | 3.9 | 3.9 |
| *S. marcescens* | 131 | 3.9 | 1.0 | 1.0 | 7.8 |
| *P. stuartii* | 6570 | >250—1000 | 15.6 | 15.6 | 12.5 |

B: 3′-(R)-3′-(aminomethyl)dihydrospectinomycin
C: 3′-(R)-3′-(N-aminomethyl)dihydrospectinomycin
D: 3′-(R)-3′-(N-octylaminomethyl)dihydrospectinomycin

Antimicrobial compounds within the scope of the present invention are active against *E. coli* and can be used, for example, to reduce, arrest and eradicate slime production in papermill systems caused by this microorganism. The antimicrobial compounds can also be used to prolong the life of cultures of *Trichomonas foetus*, *Trichomonas hominis* and *Trichomonas vaginalis* by freeing them of *E. coli* contamination. The antimicrobial compounds can be used to swab laboratory benches and equipment in mycological laboratories. The antimicrobial compounds can also be used effectively against *K. pneumoniae*.

To be used as suggested above, the antimicrobial compounds of the present invention are incorporated into solutions, powders, suspensions, water-in-oil emulsions and like means of delivery which are well known and are described in greater detail below in connection with administration to mammals and humans. The concentration of antimicrobial compound in these means of delivery or application must be at least sufficient to inhibit the growth of target microorganisms at site of application. These minimum effective concentrations will vary, depending on the target microorganism, the environment in which its growth is to be slowed or it is to be eliminated, and the particular means of delivery used. The minimum effective concentration could be determined readily by a person skilled in the art. It is contemplated that the minimum effective concentration will range from about 1 part per million to about 10,000 parts per million, preferably 10 parts per million to 1000 parts per million in the solution, powder, suspension or emulsion. The preferred carrier is water.

The antimicrobial compounds within the scope of the present invention are useful for treating or preventing microbial infections, particularly bacterial infections, in mammals, including humans, suffering from or susceptible to such infections. The anti-microbial activity of a compound in mammals, including humans, is suggested by a minimum inhibitory concentration in the in vitro test described above of less than about 50 µg/ml against a microorganism. The antimicrobial activity of a compound against a microorganism in mammals, including humans, is ascertained more definitely by testing the ability of the compound to cure mammals, such as mice, rats or rabbits, which have been challenged with an acute dose of the microorganism.

The compounds of formula I are also effective for treating bacterial infections, such as gonorrhea, in mammals, including humans.

The compositions of the present invention are presented for administration to humans and animals in unit dosage forms, such as tablets, capsules, pills, powders, granules, sterile parenteral solutions or suspension, eye drops, oral solutions or suspensions, and water-in-oil emulsions containing suitable quantities of the compound of formula I.

For oral administration, either solid or fluid unit dosage forms can be prepared. For preparing solid compositions such as tablets, the compound of Figure 1 is mixed with conventional ingredients such as talc, magnesium stearate, dicalcium phosphate, magnesium aluminum silicate, calcium sulfate, starch, lactose, acacia, methylcellulose, and functionally similar materials as pharmaceutical diluents or carriers. Capsules are prepared by mixing the compound with an inert pharmaceutical diluent and filling the mixture into the hard gelatin capsule of appropriate size. Soft gelatin capsules are prepared by machine encapsulation of a slurry of the compound with an acceptable vegetable oil, light liquid petroleum or other inert oil.

Fluid unit dosage forms for oral administration such as syrups, elixirs, and suspensions can be prepared. The water-soluble forms can be dissolved in an aqueous vehicle together with sugar, aromatic flavoring agents and preservatives to form a syrup. An elixir is prepared by using a hydroalcoholic (ethanol) vehicle with suitable sweeteners such as sugar and saccharin, together with an aromatic flavoring agent.

Suspensions can be prepared with an aqueous vehicle with the aid of a suspending agent such as acacia, tragacanth, methyl-cellulose and the like.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilized before filling into a suitable vial or ampoule and sealing. Advantageously, adjuvants such as a local anesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection is supplied to reconstitute the liquid prior to use. Parenteral suspensions can be prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

Additionally, a rectal suppository can be employed to deliver the active compound. This dosage form is of particular interest where the mammal cannot be treated conveniently by means of other dosage forms, such as orally or by insufflation, as in the case of young children or debilitated persons. The active compound can be incorporated into any of the known suppository bases by methods known in the art. Examples of such bases include cocoa butter, polyethylene glycols (Carbowaxes), polyethylene sorbitan monostearate and mixtures of these with other compatible materials to modify the melting point or dissolution rate. These rectal suppositories can weigh from about 1—2.5 gm.

The term "unit dosage form" as used in the specification refers to physically discrete units suitable as unitary dosages for human subjects and animals, each unit containing a predetermined quantity of active material calculated to produce the desired therapeutic effect in association with the required pharmaceutical diluent, carrier or vehicle. The specifications for the novel unit dosage forms of this invention are dictated by and directly dependent on (a) the unique characteristics of the active material and the particular effect to be achieved and (b) the limitations inherent in the art of compounding such an active material for use in humans and animals, as disclosed in detail in this specification, these being features of the present invention. Examples of suitable units dosage forms in accord with this invention are tablets, capsules, pills, suppositories, powder packets, wafers, granules, cachets, teaspoonfuls, tablespoonfuls, dropperfuls, ampoules, vials, aerosols, with metered discharges, segregated multiples of any of the foregoing, and other forms as herein described.

An effective quantity of the compound is employed in treatment. The dosage of the compound for treatment depends on many factors that are well known to those skilled in the art. They include, for example, the route of administration and the potency of the particular compound. A dosage schedule for humans of from 2 to 5000 mg of compound in a single dose, administered parenterally or in a composition, is effective for treating bacterial infections. The single dose is preferably from 5 to 5000 mg, and more preferably 10 to 2500 mg.

Preferred anti-microbial agents within the scope of the present invention are compounds of formula I wherein R is $C_{1-4}$ alkyl, one of $A_1$ and $A_2$ is OH and the other is $CH_2NHR_8$, especially when the configuration at C—3′ is (R), and wherein $R_8$ is $C_{1-8}$ n-alkyl or cyclohexylmethyl. Especially preferred are such compounds wherein R is methyl.

The preparation of compounds of the invention will first be illustrated with reference to Charts A to E. Compounds of formula $III_{(b)}$ are the starting material for Charts A, D and E, and this and other compounds differing only in the C—3′ substitution are represented in the Charts solely by an indication of the substituent(s) at "3′C".

The Roman numeral identifying each formula herein may have the subscript "b" to indicate that the nitrogen atoms in the spectinomycin compound (other than at C—3′) are blocked. The subscript "(b)" indicates that blocking is optional (although preferred). Active compounds of the invention (represented by Roman numerals without any subscript) are obtained from the corresponding blocked compounds by deblocking.

All compounds of formula III are known; see US—A—4351771. A compound of formula $III_b$ may be

prepared by introducing a N-blocking group into $R_3$ by the procedures exemplified in Examples below for blocking the actinamine-ring nitrogen-blocked spectrinomycin-analogue starting material wherein $R_3$ is an aminoalkyl or substituted aminoalkyl group. After this blocking reaction, wherein the starting material is unblocked, $R_3$ will be the same as the blocking groups on the actinamine-ring nitrogens. If the starting material spectinomycin analogue is blocked before use in the reaction to block a nitrogen optionally present in $R_3$, that blocking group can be different from the blocking groups on the actinamine-ring nitrogens.

"Blocking groups", including those optionally present on the nitrogen atom, if any, in the substitutent on C—6', are selected from a large number of such groups well known from peptide chemistry. Columns 2 and 3 of US—A—4173647 list all these which can be used in the present invention as well as several (4-acetoxy- or 4-ethoxycarbonyloxybenzyloxycarbonyl, alkoxycarbonyl substituted by 2-furyl- or p-toluenesulfonyl, phenoxycarbonyl substituted by nitro, and any dialkylaminooxycarbonyl) which are not suitable for use in the present invention. Procedures for the introduction and removal of suitable blocking groups will be readily understood by those skilled in the art. The preferred blocking group in all reactions described herein is carbobenzyloxy.

Except as otherwise noted herein, the deblocking steps indicated in the various Charts are required to obtain anti-microbially active compounds from blocked intermediates. Deblocking simply replaces blocking groups on the nitrogen atoms to which they are bonded by hydrogen atoms, and does not effect the molecules in any other way.

When deblocking leads directly to an acid addition salt, the free base can be generated from such salt by methods well known in the art, e.g. passing a solution of the salt in a solvent such as water, methanol, ethanol, tetrahydrofuran or 1,2-dimethoxyethane through a basic ion-exchange resin, and collected fractions of eluate which contain the free base.

Pharmacologically-acceptable acid addition salts can be produced from the free bases by methods well known in the art, e.g. neutralising the free base in solution in a solvent such as water, methanol, ethanol, isopropanol, ether, 1,2-dimethoxyethane or p-dioxane with any acid which has a pharmacologically-acceptable conjugate base, and then isolating the salt by filtration and direct crystallisation or by evaporation of solvent followed by subsequent recrystallisation from a suitable solvent. Acids which have pharmacologically-acceptable conjugate bases include hydrochloric, hydrobromic, hydroiodic, sulfuric, phosphoric, maleic, citric, tartaric, fumaric and acetic acids; hydrochloric acid is preferred.

Referring to Chart A, a formula $IV_b$ compound is obtained by reaction of the formula $III_b$ compound with hydrazine ($R_{60}$ = H) or, preferably, an arylsulfonylhydrazine ($R_{60} = ArSO_2$). The method is as described in US—A—4173647 and DE—A—2756912. Ar can be phenyl optionally substituted by one or two $C_{1-6}$ alkyl, $C_{3-8}$ cycloalkyl or phenyl likewise optionally substituted by one or two $C_{1-6}$ alkyl groups. Ar is preferably phenyl, o-tolyl, m-tolyl or, most preferably, p-tolyl.

A formula $V_b$ compound is obtained either by reaction of the formula $IV_b$ ($R_{60}$ = H) compound with an oxidising transition metal oxide such as $MnO_2$, $Ag_2O$ or HgO, or by reaction of a formula $IV_b$ ($R_{60} = ArSO_2$) compound with an organic amine base, preferably triethylamine. Preferably, twice as much of the base as the formula $IV_b$ arylsulfonylhydrazone is used. Either reaction is conducted in any organic solvent which is inert with respect to the reactants and in which the reactants are soluble. For the formation of the formula $V_b$ diazo ketone from the formula $IV_b$ arylsufonylhydrazone, examples of such solvents are benzene, hexane, dimethyl ether, diethyl ether, tetrahydrofuran and chlorinated hydrocarbons such as methylene chloride and ethylene chloride. The preferred solvent is methylene chloride.

The $III_b \rightarrow V_b$ transformation can be carried out at any temperature at which it proceeds at an acceptable rate, at which the reactants remain in solution in the solvent being employed, and at which reactants or products do not thermally decompose. Generally, temperatures between 0 and 60°C are acceptable. Preferred temperatures are 20—30°C.

A formula $VI_b$ compound is obtained by reacting a formula $V_b$ compound with a salt of the formula $CH_2{=}NR_5R_6\,X^-$ in the presence of a tertiary amine base, preferably triethylamine, and a CN-bearing solvent, preferably acetonitrile. The salts are known or are easily synthesised on the basis of the teachings of Schreiber *et al*, Agnew. Chem. (Int'l Ed., English) *10* (1971) 330, and Kinast *et al*, Agnew. Chem. (Int'l Ed., English) *15* (1976) 239. It is important in carrying out the reaction that as little water as possible is present in the reaction medium. Thus, the solvent and the amine base should be dry; the salt and the compound of formula $V_b$ should be as dry as possible, the reaction vessel should be dried before the introduction of reactants, and the vessel should be flushed with a stream of unreactive, dry gas (e.g. $N_2$) during the reaction. Those of skill in the art will be familiar with methods to minimise the amount of water in a reaction medium. The reaction can be carried out at any temperature at which reactants and products are soluble in the solvent used and are stable, and at which the reaction proceeds at an acceptable rate, preferably 20 to 30°C.

The mixture of formula $VII_b$ and $VIII_b$ compounds is preferably obtained by reaction of a formula $VI_b$ compound with $Na[AlH_2(OCH_2CH_2OCH_3)_2]$, followed by quenching with water then ethyl acetate. An alternative procedure comprises reaction with $NaCNBH_3$ under mildly acidic conditions (pH ~ 4), followed by quenching with a weak acid (pKa ≧ 6).

In the compounds of formula $VI_b$, the configuration at the vinylogous carbon bonded to C—3', with respect to C—2', is not known. However, the process $V_b \rightarrow VI_b$ illustrated in Chart A produces a compound

stereospecifically at that position. Thus, the configuration is E or Z, but not both.

Similarly, in compounds of formula $VIII_b$, the configuration at C—3′ is not known, although it is known that the process $VI_b \rightarrow VIII_b$ illustrated in Chart A is stereospecific at C—3′. Thus, the compound of formula $VIII_b$ has configuration 3′-(S) or 3′—(R), but not both.

With reference to Charts B and C, formulae $IX_b$ and $X_b$ compounds are obtained by the non-stereospecific alkaline epoxidation of a formula $VII_b$ compound, using $H_2O_2$. The $IX_b \rightarrow XI_b$ and $X_b \rightarrow XII_b$ conversions each involve reaction with $N_3^-$ in the presence of $NH_4^+$. The $IX_b \rightarrow XV_b$ and $X_b \rightarrow XVI_b$ conversions each involve reaction with $CN^-$ in the presence of $NH_4^+$. THe $IX_b \rightarrow XIII_b$ and $X_b \rightarrow XIV_b$ conversions each involve reaction with $R_1R_2NH$.

With reference to Charts D and E (in which $R_2'$ is the same as $R_2$ but is not hydrogen), $XVIII_{(b)} \rightarrow XVII_{(b)}$ isomerisation can be achieved by reaction with a base or basic ion-exchange resin, in an inert organic solvent. The $III_{(b)} \rightarrow XVII_{(b)}$ and $III_{(b)} \rightarrow XVIII_{(b)}$ conversions are hydrocyanation processes of the invention generally described above. The preferred temperature for these processes is 20—30°C.

The hydrocyanation reactions involve the use of any compound which, under the basic reaction conditions, in the unreactive solvent employed, will produce by, for example, decomposition or dissociation, a cyano group which can react with the 3′ carbon of the spectinomycin or spectinomycin-analogue starting material. The preferred compound is acetone cyanohydrin.

For both the hydrocyanation and the isomerisation, the base which may be used is preferably triethylamine or potassium or sodium carbonate. The "basic ion-exchange resin" which may be used is an ion-exchange resin wherein the ion-exchange groups are basic in character, i.e. acceptors of protons from, or donors of electron pairs to, compound which pass or diffuse through the resin. Examples of basic ion-exchange resins wherein the ion-exchange groups are tertiary amino groups include Amberlite IRA—45, IRA—68, IRA—93 and IRA—94, and Amberlyst A—21, all available from the Rohm and Haas Co. of Philadelphia, Pennsylvania, USA, Dowex 3—X′4, available from the Dow Chemical Co. of Midland, Michigan, USA, and Duolite A—2, A—6, A—4F and A—7 available from the Diamond Shamrock Corp. of Cleveland, Ohio, USA. Amberlite IRA—45 is the preferred resin.

In the isomerisation reaction, and the hydrocyanation reaction for the preparation of more 3′(S) than 3′(R) epimer, potassium carbonate and methanol (as solvent) are preferably used. In the hydrocyanation reaction for the preparation of more 3′(R) than 3′(S) epimer, Amberlite IRA—45 resin, methanol (as solvent), and an at least two-fold molar excess of acetone cyanohydrin are preferably used.

Removal of basic ion-exchange resin, or its neturalisation with acid, or neutralisation of base, if only base is used, slows the R to S isomerisation drastically. "Quenching" means slowing sufficiently to permit the isolation of the R epimer and its stabilisation by, for example, further reaction, before the ratio of the amount of R epimer to the amount of S epimer becomes less than 1.

If required, $XVII_b \rightarrow XVII$ and $XVIII_b \rightarrow XVIII$ deblocking reactions involve Pd black with HCOOH.

The $XVII_{(b)} \rightarrow XIX_{(b)}$ and $XVIII_{(b)} \rightarrow XX_{(b)}$ conversions involve $PtO_2/H_2$ or Raney nickel/$H_2$ in the presence of acetic acid. $XI_b \rightarrow XIX_b$ and $XII_b \rightarrow XX_b$ conversions involve hydrogenation over $PtO_2$. $XI_b$ and $XII_b$ can be converted directly to unblocked XIX and XX, respectively, by reaction first with Pd black/HCOOH and then with HCl.

The $XIX_b \rightarrow XXI_b$ and $XX_b \rightarrow XXII_b$ conversions involve reductive alkylation (blocking is essential). The $XIX_{(b)} \rightarrow XXIII_{(b)}$ and $XX_{(b)} \rightarrow XXIV_{(b)}$ conversions involve acylation by known methods such as reaction with an acid anhydride, with an acid chloride, in the presence of base, or with an acid and a coupling reagent such as dicyclohexylcarbodiimide or an alkyl chloroformate.

Whenever synthetic methods disclosed herein result in two or more compounds related as stereoisomers, such compounds will also be related as diastereomers and consequently will have differing physical and chemical properties. The different properties can be used to separate the stereoisomers from each other by any of numerous techniques known in the art.

The following Examples illustrate how compounds of the invention may be prepared.

Unless otherwise specified, specific rotations reported in the Examples are measured at room temperature, 24±2°C. Gram-molecular weights reported in the Examples are determined by high resolution mass spectrometry using, when necessary, trimethylsilated compounds. The extent of silation is indicated for each Example. A designation "M(+−Q)", wherein W is the formula for a functional group or other molecular fragment indicates that the reported mass is determined from the mass of the molecular ion (M+) missing molecular fragment or function group Q.

## Example 1

N,N-dicarbobenzyloxyspectinomycin tosylhydrazone (Formula $IV_b$: $R_{60}$ is tosyl, $R_7$ is carbobenzyloxy and R is methyl)

Refer to Chart A.

In 40 ml of absolute ethanol are dissolved 2.5 g (4.2 mmol) of N,N′-dicarbobenzyloxyspectinomycin (formula VIII) and 0.77 g (4.2 mmol) of p-toluenesulfonyl hydrazide. The reaction is stirred for 2 hr at room temperature under $N_2$. At this time TLC shows no remaining N,N′dicarbobenzyloxyspectrinomycin. Removal of the solvent in vacuo affords 3.13 g (98%) of title product as a white solid foam, which is used without further purification: CMR ($d_6$-Acetone) δ 167.6, 153.4, 144.6, 137.9, 136.4, 130.1, 129.9, 129.0, 128.3, 97.3, 89.9, 74.7, 69.0, 67.2, 66.4, 66.0, 57.70, 57.5, 53.5, 33.2, 31.7, 31.4, 31.2 and 21.3.

Example 2

N,N'-dicarbobenzyloxy-3'-deoxo-3'-diazosecospectinomycin (Formula $V_b$: $R_7$ is carbobenzyloxy and $R_3$ is methyl)

Refer to Chart A.

In a 500 ml round-bottomed flask are dissolved 13.16 g (17.1 mmol) of N,N'-dicarbobenzyloxy-spectinomycin tosylhydrazone (Example 1) in 300 ml of $CH_2Cl_2$. To this solution are then added 2.4 ml (17.1 mmol) of triethylamine. The solution immediately turns yellow and is stirred under $N_2$ for 5 hr. Removal of the solvent in vacuo affords 14.25 g of crude title products as yellow, brittle foam. The product is chromatographed on 1 kg of silica gel slurry-packed with ethyl acetate. The column is eluted with ethyl acetate. The elution sequence is an follows: 750ml ethyl acetate, nil; 2450 ml ethyl acetate, 4.75 g of starting tosylhydrazone; 4900 ml ethyl acetate, 6.29 g of diazoketone. The yield of product based on recovered starting material is 94.2%. The product is obtained as a yellow solid: mp 84—90° (decomp); IR (KBr), 3450 (a), 2950 (m), 2100 (s), 1675 (s) $cm^{-1}$; PMR ($CDCl_3$) δ 1.38 ($CH_3CH$), 2.7, 3.1 ($CH_2$), 3.2 ($CH_3$—N) 3.2 (CH—N) 3.2 (CH—N, CH—O), 5.1 (anomeric, $OCH_2Ph$) and 7.3 (aromatic); CMR (Acetone-$d_6$) δ 189.5, 157.5, 138.0, 129.0, 128.2, 102.1, 92.2, 74.5, 73.8, 69.2, 67.9, 67.11, 64.2, 60.1, 59.8, 59.5, 59.3, 31.8, 31.7, 31.46, 31.3, 30.7, 29.8, 29.2, 28.8 and 20.9; $[\alpha]_D$ = −25° (c, 7495, $CHCl_3$); UV ($CH_3CN$) λ (max) 297 μ (ε=8181); M.S. no M+ observed.

| | | | |
|---|---|---|---|
| Anal. calc'd for $C_{30}H_{36}O_{10}H_2$: | C, 58.82; | H, 5.92; | N, 9.15. |
| Found: | C, 55.84; | H, 5.79; | N, 7.03. |

When this reaction is repeated using 2 equivalents of triethylamine, N,N'-dicarbobenzyloxy-3'-deoxo-3'-diazospectinomycin is isolated in 75% yield.

Example 3

N,N-di-tert-butoxycarbonylspectinomycin (Formula $III_6$: $R_7$ is tert-butoxycarbonyl and R is methyl)

Refer to Chart A

In 700 ml of $H_2O$ contained in a 3 l Morton flask with overhead stirrer and addition funnel are dissolved 200 g 0.40 mol) of spectinomycin sulfate tetrahydrate. To this solution are carefully added 64 g (0.80 mol) of $NaHCO_3$ in batches. After stirring for 0.5 hr, 700 ml of t-butanol are added, followed by the addition of 176.6 g (0.81 mol) of di-t-butyl-dicarbonate over a period of 15 min. The reaction is then stirred for 65 hr at room temperature. The reaction is then transferred to a 4 l separatory funnel and the aqueous phase is separated from the tert-butanol. The tert-butanol is removed in vacuo using a dry ice condenser under high vacuum. The residue consists of a gum which is taken up in 50 ml of ethyl acetate. The aqueous phase is concentrated and extracted with ethyl acetate (2 × 300 ml). All of the ethyl acetate solutions are combined and washed with water (1 × 500 ml). An emulsion forms which is broken up with the addition of 500 ml of brine. The aqueous wash is backwashed with 200 ml of ethyl acetate and the combined organics are washed with brine (2 × 500 ml) and dried over $Na_2SO_4$. The solvent is removed in vacuo and the product is placed under high vacuum overnight to afford 131.38 g (62%) title product of a white solid. No residual tert-butanol is seen by $C^{13}$ NMR. The title product is used without further purification: IR ($CHCl_3$) 3420, 1740 (s), 1675 (s), 1365 (s), 1255 (s), 1160 (s), 1130 (s), 1060 (s), and 885 $cm^{-1}$; NMR ($CDCl_3$) δ 1.4 $(CH_3)_3$ C—O), 1.4 ($CH_3$—CH), 2.0—3 ($CH_2CO$, CHN's), 2.9, 3.0 ($CH_3N$'s), 3.1—5.0 (CH—O's); CMR δ 97.8, 79.5, 75.1, 68.1, 66.3, 65.6, 60.4, 30.8, 29.8, 28.8, 28.5, 27.9, and 21.5; $[\alpha]_D$ = −2° (C, 0.904, $CHCl_3$).

| | | | |
|---|---|---|---|
| Anal. calc'd for $C_{24}H_{40}N_2O_{11}$: | C, 54.13; | H, 7.57; | N, 5.26. |
| Found: | C, 52.8; | H, 7.41; | N, 4.76. |

Exact mass calc'd for $C_{33}H_{64}N_2O_{11}Si_3$: 748.3818;
Found 748.3772

Example 4

N,N'-di-tert-butoxycarbonylspectrinomycin tosylhydrazone (Formula $IV_b$: $R_{60}$ and R are as in Example 1 and $R_7$ is as in Example 3)

Refer to Chart A.

A solution containing 100 g (0.19 mol) of N,N'-di-tert-butoxycarbonyl-spectinomycin and 35 g (0.19 mol) of p-toluenesulfonylhydrazide in 475 ml of absolute ethanol is stirred for 6.5 hr under $N_2$ at room temperature. The solvent is removed in vacuo and the product is placed under high vacuum overnight. This affords 131 g (100%) of a white solid which is used without further purification. IR (KBr) 3500 (s), 2950 (s), 1650 (s), 1340 (s, broad), 1120 (s, broad), 1090 (s, broad), 925 (s) and 885 (s) $cm^{-1}$; $[\alpha]_D$ = −2° (C, 0.982, $CHCl_3$); PMR ($CDCl_3$) δ 1.4 (tButyl), 2.8—3.0 ($CH_3N$'s), 3.4—4.8 (CH—O's), 7.3, 7.8 (aromatic). CMR ($d_6$-Acetone) δ 153.7, 130.2, 129.8, 128.6, 97.4, 90.0, 79.9, 74.9, 69.1, 66.4, 57.7, 30.8, 30.7, 29.8, 28.9, 28.5, 27.9, 21.3 and 18.6.

| | | | | |
|---|---|---|---|---|
| Anal. calc'd for $C_{31}H_{48}N_4O_{12}S$: | C, 53.13; | H, 6.90; | N, 7.99 | S, 4.58. |
| Found: | C, 51.85; | H, 6.90; | N, 7.67. | |

Example 5

N,N′-di-tert-butoxycarbonyl-3′-deoxo-3′-diazo-secospectinomycin (Formula $V_b$: $R_7$ is tert-butoxycarbonyl and R is methyl)

Refer to Chart A.

To a solution containing 50 g (71.3 mmol) of N,N′-di-tert-butoxycarbonyl-spectinomycin tosylhydrazone in 500 ml of $CH_2Cl_2$ are added 15 ml (107.6 mmol) of triethylamine. The solution turns yellow/orange and is stirred at room temperature under $N_2$. During the course of the reaction an additional 10 ml (71.3 mmol) of triethylamine is added. After stirring for 8 hr at room temperature, the reaction is stored in the freezer overnight. The next morning the reaction is concentrated to a volume of 100 ml and chromatographed on 1.2 kg of silica gel which has been slurry packed in $CH_2Cl_2$ and then the column was eluted with ethyl acetate. Chromatography affords 33.09 g (85%) of N,N′-di-tert-butoxycarbonyl-3′-deoxo-3′-diazospectinomycin as a yellow solid; IR (Kbr) 3350 (s) 2850 (s), 2050 (s), 1625 (s), 1400 (s), 1325 (s), 1220 (s), 1125 (s), 1030 (s) and 885 (s) $cm^{-1}$; CMR ($D_6$-Acetone) δ 189.3, 102.17, 92.7, 79.32, 74.3, 69.1, 68.0, 67.1, 64.0, 59.7, 59.6, 59.2, 30.8, 30.6, 29.9, 29.2, 28.9, 28.6, 27.9 and 21.0; $[\alpha]_D$ = 226° (C, 1.095, $CHCl_3$).

Anal. calc'd for $C_{24}H_{40}N_4O_{10}$: C, 52.93; H, 7.40; N, 10.29.
Found: C, 51.96; H, 7.25; N, 7.48%.

Example 6

N,N′-dicarbobenzyloxy-3′-deoxo-3′-diazosecospectinomycin (as Example 3)

Refer to Chart A.

In 10 ml of $CHCl_3$ are dissolved 500 mg (0.814 mmol) N,N′-dibenzyloxycarbonylspectiomycin hydrazone. To this solution are added 500 mg (5.75 mmol) of active $MnO_2$. The resulting slurry is stirred for 24 hr at room temperature. The mixture is filtered through a pad of magnesium silicate and the filtrate is concentrated to dryness in vacuo to afford 258 mg of a yellow solid. The product is taken up in ethyl acetate and chromatography on 20 g of silica, slurry-packed in ethyl acetate. The column is eluted with ethyl acetate (2 l). In elution volume 0—200 ml there are obtained 180 mg of a mixture of title product and rearranged product. Elution volume 200—550 ml contains 63 mg of pure title product (12.6% yield) whose $^{13}C$—NMR is identical with that obtained from the treatment of N,N′-dibenzyloxycarbonylspectinomycin tosylhydrazone with triethylamine.

Example 7

3′-(Dimethylamino)methylene-N,N′-dicarbobenzyloxy-secospectinomycin (Formula $VI_b$: $R_7$ is carbobenzyloxy and R, $R_5$ and $R_6$ are methyl)

Refer to Chart A.

A 100 ml Morton flask is flame-dried under a stream of nitrogen. After cooling, the flask is charged with 2.0 g (21.4 mmol) of N,N-dimethylmethylene ammonium chloride and 50 ml of acetonitrile. To this suspension are added 1.7 ml (23.1 mmol) of dry $Et_3N$ followed by 5.0 g (8.2 mmol) of N,N′-dicarbobenzyloxy-3′-deoxo-3′-diazo-secospectinomycin (Example 2). The solution is stirred for 4.5 hr at room temperature under nitrogen. During this time a slow evolution of nitrogen is observed, the yellow color of the starting material fades and a white precipitate forms. The reaction mixture is filtered and the filtrate concentrated in vacuo to afford 6.73 g of a yellow solid. The product is taken up in $CHCl_3$ and chromatographed on 250 g of silica slurry packed in $CHCl_3$. The column is eluted with an acetone/$CHCl_3$ gradient as follows with 50-ml fractions being collected: 500 ml, 10% acetone/$CHCl_3$; 500 ml 20% acetone/$CHCl_3$; 5 liter 30% acetone/$CHCl_3$ and 1 liter 40% acetone/$CHCl_3$. Fractions 50—133 contain title product. These fractions are pooled and the solvent is removed in vacuo to afford 3.14 g (60%) of product: $^{13}$—NMR ($d_6$-Acetone) δ 190.0, 154.1, 138.2, 129.1, 128.3, 128.0, 102.6, 99.4, 91.6, 74.5, 69.7, 68.1, 67.9, 67.2, 60.1, 43.6, 33.3, 31.8, and 21.4; $^1H$ NMR ($CDCl_3$) δ 1.32 ($CH_3CH$, d, J=6Hz) 3.11 (N—$CH_3$'s) 3.3—4.4 (OCH, NCH), 5.1 (anomeric, $OCH_2Ph$) 7.25 (aromatic) and 7.7 (=CH—$N(CH_3)_2$); $[\alpha]_D$ +64 (C, 0.998, $CHCl_3$); UV max (EtOH) 204 (ε, 22150) and 338 (ε 17,500); IR ($CHCl_3$) 3439 (m), 3009 (m), 2905 (m), 1691 (s), 1652 (m), 1539 (s), 1485 (s), 1453 (s), 1421 (s), 1385 (s), 1343 (s), 1268 (s), 1171 (s) and 1024 $cm^{-1}$.

Exact mass calc'd for $C_{39}H_{57}N_3O_9Si_2$ (tri—O—TMS—TMSOH): 767.3633;
Found: 767.3648.

Example 8

N,N′-dicarbobenzyloxy-3′-deoxo-3′-methylenespectinomycin (Formula $VII_b$)

Refer to Chart A.

In 10 ml of methanol are dissolved 2.0 g (3.1 mmol) of Example 7 product. To this solution is added a trace of bromcresol green indicator in methanol. The solution is adjusted to pH ≤4 by the addition of 0.1N methanolic HCl and 65 mg (1.03 mmol) of $NaCNBH_3$ in 1 ml of methanol is added. The pH is kept near 4 and the reaction stirred for 2.5 hr. The reaction is then quenched by the addition of 5 ml of saturated $NaHCO_3$ and the methanol is removed in vacuo. The residue is partitioned between 20 ml of water and 20 ml of ethyl acetate. The ethyl acetate is separated and combined with a 10 ml ethyl acetate extract of the aqueous phase. The combined extracts are washed with 20 ml of brine and dried over $Na_2SO_4$. Removal of the solvent in vacuo affords 1.73 g of a blue solid. The product is dissolved in $CHCl_3$ and chromatographed on 200 g of silica gel, slurry packed in $CHCl_3$. The column is eluted with an acetone/$CHCl_3$ gradient as follows:

400 ml 10% acetone in $CHCl_3$; 400 ml 20% acetone in $CHCl_3$; 2 liter 30% acetone in $CHCl_3$; 1 liter 1:1 acetone/$CHCl_3$. Elution volume 1400—1650 ml contained 245 mg of over-reduced product. Elution volume 1750—2300 ml contains 439 mg of N,N'-dicarbobenzyloxy-3'-deoxo-3'-methylenespectinomycin, title product, as a white solid. Elution volume 3500 ml finish contains 167 mg of amino compound 3'-deoxo-3'-(N,N-dimethylaminomethyl)-dihydrospectinomycin. The yield of desired methylene compound is 23%: $^{13}C$—NMR ($d_6$-Acetone) δ 146.0, 138.3, 129.1, 128.3, 112.1, 97.5, 92.1, 74.5, 74.0, 72.9, 67.2, 66.4, 65.2, 60.3, 57.8, 57.7, 57.5, 40.8, 31.6 and 21.3; $^1H$—NMR ($CDCl_3$) δ 1.2 ($CH_3CH$, d, J=6Hz), 2.0—2.5 (CH, $CH_2$), 2.9 ($NCH_3$'s), 3.8—4.4 (CH—O, CH—N), 4.5 ($CH_2$=), 5.1 (anomeric, $OCH_2Ph$) and 7.2 (aromatic); IR ($CHCl_3$) 3673 (w), 3587 (m), 3418 (s), 3009 (s), 2952 (s), 1682 (s), 1486 (s), 1454 (s), 1407 (s), 1386 (s), 1346 (s), 1264 (s), 1234 (s), 1167 (s), 1128 (s), 962 (m) and 918 (m) $cm^{-1}$; $[\alpha]_D = -6°$ (C, 1,042, $CHCl_3$).

Exact mass calc'd for $C_{30}H_{57}O_{10}N_2Si_2$ (M+ —$CH_3$ for the bis TMS deriv): 727.3026;
Found: 727.3069.

## Example 9

N,N'-dicarbobenzyloxy-3'-deoxo-3'-methylenespectinomycin and N,N'-dibenzyloxycarbonyl-3'-deoxo-3'-(N,N-dimethylaminomethyl)dihydrospectinomycin

Refer to Chart A.

A 300 ml 3-necked Morton flask is flame-dried under a stream of nitrogen. After cooling, the flask is charged with 5.0 g (7.79 mmol) of the enaminone product of Example 7 and 50 ml of freshly distilled anhydrous tetrahydrofuran. To this solution are then added 2.4 ml of a 3.5M solution of $NA[AlH_2(OCH_2CH_2OCH_3)_2]$ in toluene, via syringe over a period of 15 min. A vigorous evolution of gas is observed. At completion of the addition, TLC shows a complete loss of starting material. The reaction is then quenched by adding 100 ml of water and 100 ml of ethyl acetate. The entire mixture is then poured into 300 ml of ethyl acetate. The aqueous phase is separated and combined with 2 × 100 ml water washes of the ethyl acetate. The combined aqueous washes are re-extracted with ethyl acetate (2 × 50 ml). The combined ethyl acetate extracts are washed with 200 ml of brine and dried over $NaSO_4$. After filtering, the solvent is removed in vacuo to afford 4.89 g of a white solid. The product is taken up in $CHCl_3$ and chromatographed on 300 g of silica, slurry-packed in $CHCl_3$. The column is eluted as follows: 4 l 1% methanol/$CHCl_3$; 4 l 1.5% methanol/$CHCl_3$; 6 l 2% methanol/$CHCl_3$; and 2 l 10% methanol/$CHCl_3$. The first 3 l of eluant are collected and then 50 ml fractions are taken. In elution volume 5.25 l to 9.45 l, there appears 1.75 g (36% of theor.) of the title methylene compound. In elution volume 9.5 l to the finish, 1.954 g of a mixture of methylene and amino title compounds is obtained.

## Example 10

N,N'-dibenzyloxycarbonyl-3'-deoxo-3'-methylenespectinomycin oxide (Formula $IX_b$)

Refer to Chart B.

In 10 ml of $CH_2Cl_2$ is dissolved 1.0 g (1.67 mmol) of N,N'-dibenzyloxycarbonyl-3'-deoxo-3'-methylene-spectinomycin. To this solution are added 20 mg (0.075 mmol) of $VO(acac)_2$, i.e. vanadium (IV) bis(2,4-pentanedionate) oxide, followed by 2.56 ml of a 0.625 M solution of tert-butyl hydroperoxide in $CH_2Cl_2$. The reaction is stirred 22 hr, and 1 ml of tert-butyl hydroperoxide solution is added along with an additional 10 mg of $VO(acac)_2$. The reaction is stirred for 5 hr at which time TLC shows complete reaction. The solution is then poured into 20 ml of 5% aqueous $Na_2SO_3$. The $CH_2Cl_2$ is separated and combined with one 20 ml $CH_2Cl_2$ extract of the aqueous phase. The combined extracts are washed with 20 ml of brine and dried over $Na_2SO_4$. The solution is filtered and the solvent is removed in vacuo to afford 1.07 g of a brown solid. The material is dissolved in $CH_2Cl_2$ and chromatographed on 10 g of silica, slurry packed in 1:1 ethyl acetate/hexane. The column is eluted with 1 liter of 1:1 ethyl acetate. In elution volume 200—1000 ml there are obtained 800 mg (78%) of the title compound as a white solid: $^{13}C$—NMR ($d_6$-Acetone) δ 138.2, 129.2, 128.5, 96.8, 90.8, 75.0, 74.6, 74.5, 69.5, 67.4, 66.5, 65.8, 61.2, 60.2, 59.3, 57.8, 57.5, 47.5, 38.4, 31.8 and 21.3. $^1H$—NMR ($CDCl_3$) δ 1.2 (d, J=6Hz, $CHCl_3$), 2.0—2.8 ($CH_2$, CH), 3.1 ($NCH_3$'s), 3.4—4.8 (OCH, NCH), 5.2 (anomeric, $OCH_2Ph$) and 7.3 (aromatic); IR ($CHCl_3$), 3412 (m), 3004 (m), 1680 (s), 1477 (m), 1450 (s), 1404 (m), 1384 (m), 1343 (s), 1241 (m), 1164 (s), 1120 (s), 1061 (s) and 886 (m) $cm^{-1}$; $[\alpha]_D = +3°$ (C, 0.9265, $CHCl_3$).

Exact mass calc'd for $C_{39}H_{59}N_2O_{11}Si_3$ (m+ —$CH_3$): 815.3426;
Found: 815.3443.

## Example 11

N,N'-dibenzyloxycarbonyl-3'-azidomethyldihydrospectinomycin (Formula $XI_b$)

Refer to Chart B.

In 30 ml of 1:5 water/EtOH are dissolved 1.139 g (1.85 mmol) of Example 26 product, 1.53 g (18.5 mmol) of $KN_3$ and 1.0 g (18.6 mmol) of $NH_4Cl$. The resulting solution is heated at 85°C for 20 min, cooled, and concentrated in vacuo. The residue is partitioned between 30 ml of ethyl acetate and 30 ml of water. The ethyl acetate is separated and combined with one 20 ml ethyl acetate extract. The combined extracts are washed with 15 ml of brine and dried over $Na_2SO_4$. The solution is filtered and the solvent removed in vacuo to afford 975 mg of a light yellow solid. The product is dissolved in $CHCl_3$ and chromatographed on 40 g of silica, slurry-packed in $CHCl_3$. The column was eluted as follows: 100 ml 1% methanol/$CHCl_3$; 1 liter 2% methanol/$CHCl_3$; 1 liter 10% methanol/$CHCl_3$. In elution volume 400—700 ml there are 427 mg of the

title compound (35%): $^{13}C$—NMR ($d_6$-Acetone) δ 138.1, 129.2, 128.8, 128.4, 94.5, 92.6, 75.3, 74.4, 73.8, 67.3, 66.4, 65.4, 61.2, 61.0, 60.4, 57.7, 57.6, 57.3, 55.7, 39.2, 31.5 and 21.1; $^1H$—NMR ($d_6$-Acetone) δ 1.2 (d, J=6Hz, $CHCH_3$) 1.3—1.9 ($CH_2$), 3.1 ($NCH_3$); 3.5—4.8 (OCH, NCH); 5.1 (anomeric, $OCH_2Ph$); 5.8 ($CH_2N_3$) and 7.3 (aromatic); IR ($CHCl_3$) 3563 (m), 3450 (m), 3015 (s), 2978 (m), 2936 (m), 2112 (s), 1690 (s), 1485 (s), 1452 (s), 1406 (s), 1383 (s), 1344 (s), 1205 (s), 1169 (s), 1124 (s), 1108 (s), 1080 (s), 1056 (s) and 723 (s) $cm^{-1}$; $[\alpha]_D = -7°$ (C, 0.994, $CHCl_3$).

Exact mass calc'd for $C_{39}H_{60}N_5O_{11}Si_3$ (M+ —$CH_3$): 858.3597;
Found: 858.3578.

Example 12

3-S-3'-aminomethyldihydrospectinomycin trihydrochloride (Formula XIX)

Refer to Chart D.

In 5 ml of methanol are dissolved 100 mg (0.152 mmol) of N,N'-dibenzyloxycarbonyl-3'-azidomethyldihydrospectinomycin (Example 11). To this solution are added 100 mg of Pd and 61 μl (1.52 mmol) of formic acid. The reaction was stirred for 10 min, was filtered and the solvent is removed in vacuo to afford 79mg of a clear glass. The product is taken up in water and 4.6 ml (0.46 mmol) of 0.1N aqueous HCl is added. The sample is frozen and lyophilized overnight to afford 62 mg (86%) of a white solid: $^{13}C$—NMR ($D_2O$) δ 93.5, 92.6, 72.5, 70.6, 68.0, 66.8, 62.3, 61.1, 59.0, 44.4, 38.8, 32.1, 31.8 and 20.6.

Example 13

N,N'-dibenzyloxycarbonyl-3'-(cyanomethyl)dihydrospectinomycin (Formula $XV_b$)

Refer to Chart B.

In 100 ml of 1:5 water/EtOH are combined 1.0 g (1.62 mmol) of Example 10 product, 1.05 g (16.2 mmol) of KCN and 866 mg of $NH_4Cl$. The reaction is stirred for 5 hr at room temperature, after which the reaction appears complete by TLC. The ethyl acetate is removed in vacuo and the residue partitioned between ethyl acetate and water. The ethyl acetate is separated and combined with one, 15 ml extract of the aqueous phase. The combined extracts are washed with 25 ml of brine and dried over $NaSO_4$. After filtering, the solvent is removed in vacuo to afford 959 mg of a white solid. The product is taken up in $CHCl_3$ and chromatographed on 100 g of silica, slurry packed in $CHCl_3$. The column is eluted as follows: 1 L 1% methanol/$CHCl_3$; 1 L 2% methanol/$CHCl_3$; 1 L 3% methanol/$CHCl_3$; 1 L 4% methanol/$CHCl_3$; 1 L 5% methanol/$CHCl_3$; 2 L 10% methanol/$CHCl_3$. In elution volume 1890 ml to 2835 ml there are obtained 254 mg of epoxide. In elution volume 3510 ml to 4410 ml there are obtained 156 mg of the title compound (19.7% yield based on recovered epoxide): $^{13}C$—NMR ($d_6$-Acetone) δ 138.2, 138.1, 129.2, 128.5, 118.2, 94.5, 92.1, 74.4, 74.2, 73.8, 73.7, 73.3, 67.3, 66.4, 65.7, 61.2, 61.1, 57.8, 57.7, 57.4, 57.3, 40.6, 31.7, 31.6, 24.9 and 20.9. Mass spec (di-TMS deriv) m/e 785 (M+) 512, 455, 299, 276, 247, 199, 149, 131, 116, 97, 85, 73 and 57.

Example 14

3'-(cyanomethyl)dihydrospectinomycin dihydrochloride

Refer to Chart B.

In 2 ml of methanol are dissolved 37 mg (0.057 mmol) of N,N'-dibenzyloxycarbonyl-3'-(cyanomethyl)-dihydrospectinomycin. To this solution are added 50 mg of Pd followed by 23 μl of formic acid. The reaction is stirred for 2 min and is filtered. Removal of the solvent in vacuo affords 25 mg of a white solid. The product is dissolved in 10 ml of 0.1N HCl and lyophilized to afford 28 mg of a white solid (100%). $^{13}C$—NMR ($D_2O$) δ 120.3, 94.0, 92.8, 73.7, 71.1, 68.7, 67.2, 62.7, 61.6, 59.4, 40.5, 32.4, 32.0, 25.5 and 20.9.

Exact mass calc'd for $C_{28}H_{59}N_3O_7Si_4$ (tetra-TMS deriv): 661.3430;
Found: 661.3407.

Example 15

N,N'-dibenzyloxycarbonyl-3-(S)-3'-aminomethyldihydrospectinomycin (Formula $XVII_b$)

Refer to Chart D.

In a Paar hydrogenation bottle are combined 406 mg (0.63 mmol) of N,N'-dibenzyloxycarbonyl-3'-(S)-3'-(azidomethyl)dihydrospectinomycin (Example 11) and 404 mg of $PtO_2$ and 10 ml of isopropanol. The solution is hydrogenated at 69 kPa (10 psi) for 1.5 hr. The solution is then filtered through magnesium silicate and the solvent removed in vacuo to afford 367 mg of a title product as a solid. The product is taken up in 5% methanol/$CHCl_3$ and filtered through 3 g of silica with 100 ml of 10% methanol/$CHCl_3$. Removal of the solvent in vacuo affords 273 mg of an off-white solid: $^{13}C$—NMR ($d_4$-methanol) 138.1, 129.5, 129.0, 128.8, 94.8, 93.7, 74.7, 73.3, 68.3, 67.7, 66.8, 65.7, 61.2, 57.9, 46.9, 40.0, 31.8 and 21.2 ppm.

Example 16

N,N'-dibenzyloxycarbonyl-3'-(R)-3'-aminomethyldihydrospectinomycin (Fromula $XX_b$)

Refer to Charts C and E.

A. In 20 ml of methanol are dissolved 1.09 g (1.67 mmol) of N,N'-dicarbobenzyloxy-3'-deoxo-3'-methylenespetinomycin (Example 8) and 531 mg (5.0 mmol) of $Na_2CO_3$. To this solution is added 0.2 ml of 30% $H_2O_2$. The reaction is stirred at room temperature for 10.5 hr, at which time TLC shows complete reaction. The reaction is poured into 50 ml of water and extracted with ethyl acetate (2 × 30 ml). The

combined extracts are washed with 30 ml of brine and dried over $Na_2SO_4$. After filtering, removal of the solvent in vacuo affords 1.027 g of a white solid. The product is taken up in $CH_2Cl_2$ and chromatographed on 75 g of silica slurry packed in 1:1 ethyl acetate/hexane. The column is eluted as follows with 30 ml factions taken: 700 ml, 1:1 ethyl acetate/hexane, 2 l 6:4 ethyl acetate/hexane and 500 ml 7:3 ethyl acetate/hexane. Elution volume 990-1680 ml contained 503 mg of a white solid which contains β-epoxide (Formula $IX_b$), approximately 1:1. Elution volume 1830 ml to 3090 ml contains 186 mg of pure α-epoxide (formula $X_b$). (18% yield): $^{13}C$—NMR ($d_6$-Acetone) 138.2, 129.1, 128.4, 96.4, 91.6, 74.9, 74.5, 69.6, 67.2, 66.4, 65.4, 60.5, 60.2, 57.5, 50.8, 38.1, 31.7 and 21.3.

B. The formula $X_b$ α-epoxide of part A (90 mg, 0.146 mmol) is dissolved in 4 ml of ethanol and potassium azide (113 mg, 1.4 mmol), ammonium chloride (53 mg, 1.0 mmol) and water (1.0 ml) are added. The mixture is stirred 5.5 hrs at 50°C under a nitrogen atmosphere, and then 16 hrs at room temperature. The solvent is then removed in vacuo and 50 ml of ethyl acetate and 5 ml of water are added. The organic layer is washed with brine and dried over magnesium sulfate. Removal of solvent in vacuo yields 93 mg (0.142 mmol, 97%) of N,N'-dibenzyloxycarbonyl-3'-(R)-3'-azidomethyl-3'-dihydrospectinomycin (formula $XII_b$) as a white solid: $^{13}C$—NMR ($d_6$-acetone) δ 157.5, 138.5, 129.2, 128.7, 128.4, 95.0, 92.9, 76.6, 74.7, 74.4, 67.5, 67.3, 66.4, 65.5, 60.9, 57.7, 54.5, 39.9, 31.8, 21.5; IR ($CHCl_3$) 3576, 3381, 3065, 3006, 2936, 2908, 2469, 2189, 1484, 1406, 1384, 1282, 1240, 1221, 1025, 1000, 951, 912, 600 $cm^{-1}$; Rf = 0.47 (silica gel, 10% methanol/chloroform).

C. Azide of part B (1.49 g, 2.27 mmol) is dissolved in 30 ml of isopropyl alcohol, 1.3 g of $PtO_2$ is added and the mixture is hydrogenated at 69 kPa (10 psi) for 2 hr. An additional 1.0 g of $PtO_2$ is added and the hydrogenation is continued for an additional 2 hr. The mixture is filtered through diatomaceous earth and magnesium silicate and solvent is removed in vacuo to afford 960 mg of a brown solid. The product is chromatographed on 40 g of 230—400 mesh silica gel packed with 1% methanol/$CHCl_3$ and eluted with 200 ml of 2% methanol/$CHCl_3$, 200 ml of 3% methanol/$CHCl_3$, 1 liter of 5% methanol/$CHCl_3$, 2 liters of 7% methanol/$CHCl_3$ and the remainder 10% methanol/$CHCl_3$, collecting 35 ml fractions. Fractions 88—126 are pooled on the basis of TLC to afford 370 mg (0.59 mmol, 26%) of title product as an off-white solid: $^{13}C$—NMR (methanol-$d_4$) δ 159, 138.0, 129.4, 128.8, 128.6, 96.2, 94.2, 74.6, 73.6, 68.1, 66.7, 65.4, 60.7, 58.0, 45.5, 41.6, 31.7 and 21.5

## Example 17

N,N'-dibenzyloxycarbonyl-3'-(R)-3'-(N-ethylaminomethyl)dihydrospectinomycin (Formula $XXII_b$)
Refer to Chart E.

In 5 ml of methanol are dissolved 591 mg (0.78 mmol) of N,N'-dibenzyloxycarbonyl-3'-(R)-3'-aminomethyldihydrospectinomycin (Example 16). To this solution is added 0.43 ml (7.8 mmol) of acetaldehyde and a trace of methyl orange indicator in methanol. Then 16.3 mg (0.26 mmol) of $NaCNBH_3$ in 1 ml of methanol are added. The pH is adjusted by addition of 1N methanolic HCl. The reaction is stirred for 1 hr and concentrated in vacuo. The residue is partitioned between water and ethyl acetate. The aqueous phase is made alkaline with concentrated $NH_4OH$ and the ethyl acetate is separated. The aqueous phase is extracted again with ethyl acetate (1 × 20 ml) and the combined extracts are washed with brine (1 × 30 ml) and dried over $MgSO_4$. After filtering, the solvent is removed in vacuo to afford 409 mg of a white solid, title product. The product is taken up in $CHCl_3$ and chromatographed on 20 gm of silica, slurry-packed in $CHCl_3$. The column is eluted as follows: 100 ml 1% methanol/$CHCl_3$; 100 ml 2% methanol/$CHCl_3$; 100 ml 3% methanol/$CHCl_3$; 100 ml 4% methanol/$CHCl_3$; 500 ml 5% methanol/$CHCl_3$; and 500 ml 7% methanol/$CHCl_3$. In elution volume 650—1000 ml there is recovered 123 mg of the title compound as a white solid: $^{13}C$—NMR ($d_6$-Acetone) 138.1, 129.1, 128.3, 128.1, 97.0, 93.8, 74.5, 71.6, 67.1, 66.4, 65.2, 57.6, 54.0, 44.4, 43.8, 31.6, 21.5 and 14.9 ppm.

Exact mass calc'd for $C_{34}H_{77}N_3O_{11}Si_4$ (tetra-TMS): 947.4635;
Found: 947.4601.

## Example 18

N,N'-dibenzyloxycarbonyl-3'-(R)-3'-(N-octylaminomethyl)dihydrospectinomycin (Formula $XXII_b$)
Refer to Chart E.

In 5 ml of methanol are combined 500 mg (0.79 mmol) of N,N'-dibenzyloxycarbonyl-3'-(R)-3'-aminomethyldihydrospectinomycin (Example 16), 1.23 ml of n-octanal and a trace of methyl orange indicator. To this solution are added 16.5 mg (0.26 mmol) of $NaCNBH_3$ in 1 ml of methanol. The pH is adjusted to 4 with 1N NCl/methanol and the reaction is stirred for 30 min. The reaction is concentrated in vacuo and the residue is taken up in ethyl acetate. The solution was washed with water (2 × 20 ml), combined with 1, 10 ml ethyl acetate backwash and washed with brine (1 × 30 ml). After drying over $MgSO_4$, the solution is concentrated to an oil. The oil is taken up in $CHCl_3$ and chromatographed on 33 g of silica, slurry packed in $CHCl_3$. The column is eluted as follows: 200 ml $CHCl_3$; 500 ml 1% methanol/$CHCl_3$; 1 liter 2% methanol/$CHCl_3$; 500 ml 3% methanol/$CHCl_3$; and 500 ml 5% methanol/$CHCl_3$. Elution volume 900—1400 ml contains 165 mg of the title compound as a white solid: $^{13}C$—NMR ($d_6$-Acetone) δ 138.2, 138.1, 129.1, 128.3, 97.0, 93.8, 74.6, 71.7, 67.1, 66.5, 65.2, 61.1, 57.6, 49.8, 44.5, 32.4, 32.1, 31.6, 30.8, 30.0, 27.8, 23.2, 21.5 and 14.3 ppm.

Exact mass calc'd for $C_{50}H_{86}N_3O_{11}Si_4$ (tetra-TMS—$CH_3$): 1016.5339;
Found: 1016.5327.

Example 19

3'-(R)-3'-(N-ethylaminoethyl)dihydrospectinomycin trihydrochloride

Refer to Chart E.

In 10 ml of methanol are dissolved 123 mg (0.19 mmol) of N,N'-dibenzyloxycarbonyl-3'-(R)-3'-(N-ethylaminomethyl)dihydrospectinomycin (Example 17). To this solution are added 123 mg of Pd black followed by 72 µl of formic acid. The reaction is stirred for 20 min, filtered and concentrated in vacuo to afford a white solid. The product is taken up in water and 1 ml of 1N HCl is added. The solution is frozen and lyophilized to afford 83 mg (89% yield) of title product, a white solid: $^{13}C$—NMR ($D_2O$) 94.0, 93.2, 73.2, 70.4, 68.2, 66.8, 66.2, 62.4, 60.5, 59.3, 50.5, 45.1, 40.7, 31.9, 31.5, 21.0 and 11.1 ppm.

Exact mass calc'd for $C_{35}H_{81}N_3O_{11}Si_6$ (hexa-TMS): 823.4690;
Found: 823.4665.

Example 20

3'-(R)-3'-(N-octylaminomethyl)dihydrospectinomycin trihydrochloride

Refer to Chart E.

In 5 ml of methanol are combined 153 mg (0.21 mmol) of N,N'-dibenzyloxycarbonyl-3'-(R)-3'-(N-octylaminomethyl)dihydrospectinomycin (Example 18) and 153 mg of Pd black. To this mixture are added 80 µl (2.1 mmol) of formic acid. The reaction is stirred for 20 min, filtered and concentrated in vacuo to afford 124 mg of a white solid. The product is taken up in water and 1 ml of 1N HCl is added. The solution is frozen and lyophilized to afford 111 mg of title product as a white solid (90% yield): $^{13}C$—NMR ($D_2O$) 91.1, 89.2, 69.2, 66.5, 64.2, 62.8, 62.2, 58.4, 56.5, 55.3, 46.9, 45.7, 36.8, 28.3, 28.2, 27.8, 27.5, 25.4, 22.9, 21.9, 19.1, 17.0 and 10.5 ppm.

Exact mass calc'd for $C_{41}H_{93}N_3O_{11}Si_6$ (hexa-TMS): 907.5629;
Found 907.5627.

Example 21

N,N'-dibenzyloxycarbonyl-3'-(S)-3'-(N-ethylaminomethyl)dihydrospectinamycin (Formula $XXI_b$)

Refer to Chart D.

In 6 ml of methanol are combined 500 mg (0.774 mmol) of N,N'-dibenzyloxycarbonyl-3'-(S)-3'-(aminomethyl)dihydrospectinomycin (Example 15), 44 µl (0.774 mmol) of acetaldehyde and a trace of methyl orange indicator in methanol. To this solution are then added 16.2 mg (0.258 mmol, 0.774 mmol $H^-$) of $NaCNBH_3$ in 1 ml of methanol. The pH is adjusted with the addition of 1N methanolic HCl until the indicator is just pink. The reaction is then stirred at room temperature and additional 1N HCl/methanol is added to keep the indicator pink. After 2.5 hr the methanol is removed and the residue partitioned between water and ethyl acetate. The aqueous phase is made alkaline with 1N NaOH and the ethyl acetate is then separated. The aqueous phase is extracted again with ethyl acetate (2 × 30 ml). The combined organics are washed with brine (1 × 50 ml) and dried over $MgSO_4$. After filtering, the solvent is removed in vacuo to afford 458 mg of a white solid. The product is taken up in $CHCl_3$ and chromatographed on 20 gm of silica gel, slurry packed in $CHCl_3$. The column is eluted as follows: 100 ml, 1% methanol/$CHCl_3$; 100 ml 2% methanol/$CHCl_3$; 100 ml 3% methanol/$CHCl_3$; 100 ml 5% methanol/$CHCl_3$; 100 ml 7% methanol/$CHCl_3$; and 700 ml 10% methanol/$CHCl_3$. In elution volume 520—720 ml there are contained 306 mg of the title compound as a white solid (60% yield): $^{13}C$—NMR ($d_6$-Acetone) 138.1, 129.1, 128.3, 94.2, 74.4, 73.1, 67.2, 66.6, 65.1, 57.6, 57.5, 57.3, 55.7, 45.0, 40.3, 31.4, 21.1 and 15.3 ppm.

Exact mass calc'd for $C_{41}H_{66}N_3O_{11}Si_3$ (tri-OTMS,—$CH_3$): 860.4005;
Found: 860.4008.

Example 22

3'-(S)-3'-(N-ethylaminomethyl)dihydrospectinomycin trihydrochloride

Refer to Chart D.

In 10 ml of methanol are combined 294 mg (0.44 mmol) of N,N'-dibenzyloxycarbonyl-3'-(S)-3'-(N-ethylaminomethyl)dihydrospectinomycin (Example 21) and 200 mg of Pd black. To this solution are added 170 µl of formic acid. The reaction is stirred for 40 mins and is filtered. Removal of the solvent in vacuo affords 225 mg of a white solid. The product is dissolved in 2 ml of water and 1.5 ml of 1N HCl is added. The solution is frozen and lyophilized to afford 215 mg of a white solid (97.5% yield): $^{13}C$—NMR ($D_2O$) 96.3, 95.1, 75.5, 74.2, 70.6, 70.5, 70.3, 65.5, 65.0, 62.2, 54.5, 47.6, 41.7, 35.3, 34.6, 23.2 and 13.79 ppm.

Exact mass calc'd for $C_{35}H_{81}N_3O_7Si_6$ (hexa TMS): 823.4690;
Found: 823.4690.

Example 23

N,N'-dibenzyloxycarbonyl-3'-(S)-3'-(N-butylaminomethyl)dihydrospectinomycin (Formula $XXI_b$)

Refer to Chart D.

In 10 ml of methanol are dissolved 507 mg (0.803 mmol) of N,N'-dibenzyloxycarbonyl-3'-(S)-3'-(aminomethyl)dihydrospectinomycin (Example 15). To this solution are added 0.7 ml of n-butyraldehyde and a drop of methyl orange indicator in methanol. This is followed by the addition of 17 mg of $NaCNBH_3$ in 1 ml of methanol. The pH is adjusted to 4 with the addition of 1N HCl/methanol. The pH is maintained at 4 for

0.5 hr while the reaction is stirred. The methanol is then removed in vacuo and the residue partitioned between ethyl acetate and water. The ethyl acetate is separated and combined with 1 × 25 ml ethyl acetate extract of the aqueous phase. The combined extracts are washed with 50 ml of brine and dried over $MgSO_4$. After filtering, removal of the solvent affords 616 mg of a white solid. The product is taken up in $CHCl_3$ and chromatographed on 30 g of silica gel, slurry packed in $CHCl_3$. The column is eluted as follows: 100 ml 1% methanol/$CHCl_3$; 100 ml 2% methanol/$CHCl_3$; 100 ml 3% methanol/$CHCl_3$; 100 ml 4% methanol/$CHCl_3$; 1.3 liter 5% methanol/$CHCl_3$. In elution volume 850—1400 ml there are recovered 250 mg of the title compound as a white solid: (45.3% yield): $^{13}C$—NMR ($d_6$-Acetone) 138.2, 129.2, 128.4, 94.2, 74.5, 73.1, 67.3, 66.7, 65.2, 57.6, 57.5, 55.9, 50.5, 40.3, 32.5, 31.6, 21.2, 20.9 and 14.2 ppm.

Exact mass calc'd for $C_{46}H_{78}N_3O_{11}Si_4$ (tetra TMS-$CH_3$): 960.4713;
Found: 960.4722.

Example 24

3'-(S)-3'-(N-butylaminomethyl)dihdyrospectinomycin trihydrochloride

Refer to Chart D.

In 10 ml of methanol are dissolved 245 mg (0.39 mmol) of N,N'-dibenzyloxycarbonyl-3'-(S)-3'-(N-butylaminomethyl)dihydrospectinomycin (Example 23). To this solution are added 250 mg of Pd black followed by 152 μl of formic acid. The reaction is stirred for 45 min and is filtered. Removal of the solvent in vacuo affords 195 mg of a white solid. The material is taken up in 2 ml of water and 1.5 ml of 1N HCl is added. The solution is frozen and lyophilized to afford 166 mg of a white solid (80% yield): $^{13}C$—NMR ($D_2O$) 96.2, 95.3, 75.6, 73.4, 70.8, 69.7, 69.5, 65.1, 63.9, 61.7, 54.9, 52.4, 41.7, 34.8, 34.6, 30.8, 23.4, 22.9 and 16.6 ppm.

Exact mass calc'd for $C_{37}H_{85}N_3O_7Si_6$ (Hexas-TMS): 851.5003;
Found: 851.4980;
Exact mass calc'd for $C_{36}H_{82}N_3O_7Si_6$ (Hexa-TMS-$CH_3$): 836.4768;
Found: 836.4757.

Example 25

N,N'-dibenzyloxycarbonyl-3'-(S)-3'-(N-octylaminomethyl)dihydrospectinomycin (Formula $XXI_b$)

Refer to Chart D.

In 5 ml of methanol are dissolved 500 mg (0.79 mmol) of N,N'-dibenzyloxycarbonyl-3'-(S)-3'-(aminomethyl)dihydrospectinomycin (Example 15). To this solution are added 1.23 ml of n-octanal and a drop of methyl orange/methanol solution. Then 17 mg of $NaCNBH_3$ in 1 ml of methanol are added. The pH is lowered to 4 with 1N HCl/methanol. The reaction is stirred for 1 hr, maintaining the pH at 4. The methanol is then removed in vacuo and the residue is taken up in $CHCl_3$. The solution is washed with 5 ml of brine and dried over $MgSO_4$. After filtering, the solvent is removed to afford an oil. The product is taken up in $CHCl_3$ and chromatographed on 30 g of silica, slurry packed in $CHCl_3$. The column is eluted as follows: 200 ml $CHCl_3$; 500 ml 1% methanol/$CHCl_3$; 1 liter 2% methanol/$CHCl_3$; 500 ml 3% methanol/$CHCl_3$; 1 liter 5% methanol/$CHCl_3$. In elution volume 1100—1400 ml there are recovered 186 mg of the title compound as a white solid (32% yield): $^{13}C$—NMR ($d_6$-Acetone) 138.2, 129.2, 128.4, 94.2, 74.5, 74.2, 74.1, 72.9, 67.3, 66.7, 66.5, 65.2, 61.4, 61.1, 60.6, 60.5, 57.6, 57.4, 55.8, 50.8, 40.4, 32.5, 31.5, 30.8, 30.3, 30.1, 29.9, 27.8, 23.2, 21.2 and 14.3 ppm.

Exact mass calc'd for $C_{51}H_{89}N_3O_{11}Si_4$ (tetra TMS): 1031.5574;
Found: 1031.5585.

Example 26

3'-(S)-3'-(N-octylaminomethyl)dihydrospectinomycin trihydrochloride

Refer to Chart D.

In 5 ml of methanol are dissolved 180 mg of N,N'-dibenzyloxycarbonyl-3'-(S)-3'-(N-octylaminomethyl)-dihydrospectinomycin. To this solution are added 200 mg of Pd black followed by 94 μl of formic acid. The reaction is stirred for 30 min and is filtered. The solvent is removed in vacuo to afford 150 mg of a white solid. The product is taken up in 2 ml of water and 0.8 ml of 1N HCl is added. The solution is frozen and lyophilized overnight to afford 135 mg of a white solid (96% yield): $^{13}C$—NMR ($D_2O$) 96.2, 95.3, 75.6, 73.4, 70.6, 69.7, 69.5, 65.1, 63.9, 61.7, 54.9, 50.7, 41.8, 34.8, 34.6, 32.0, 29.5, 28.8, 25.8, 23.4 and 17.2 ppm.

Exact mass calc'd for $C_{14}H_{93}N_3O_7Si_6$ (hexa-TMS): 907.5629;
Found: 907.5618.

Example 27

3'-(R)-3'-aminomethyldihydrospectinomycin trihydrochloride (Formula XX)

The azide of Example 16, part B, (36 mg, 0.055 mmol) is dissolved in 0.5 ml of methanol and 36 mg of palladium black are added followed by 21 μl (0.55 mmol) and formic acid. The mixture is stirred 10 min at room temperature, and filtered, rinsing the catalyst with four portions of methanol. The filtrate is concentrated in vacuo to afford a colorless material which is dissolved in 5 ml of water and treated with 0.18 ml (0.18 mmol) of 1N HCl. Lyophilization afford 30 mg of the title product as a white solid. $^{13}C$—NMR ($D_2O$, $CH_3CN$ internal reference) δ 94.3, 93.6, 73.0, 70.7, 68.4, 67.0, 66.3, 62.6, 60.7, 59.5, 43.4, 40.8, 31.9, 31.6 and 21.1.

Example 28

3'-(S)-3'-(N-propylaminomethyl)-3'-dihydrospectinomycin trihydrochloride

A. In 5 ml of freshly distilled propylamine are dissolved 93 mg (0.151 mmol) of formula $IX_b$ β-epoxide of Example 16, part A. The resulting solution is refluxed for 2 hr at which time TLC shows complete loss of starting material, with the formation of one more polar, ninhydrin active product. The propylamine is removed in vacuo and the residue is stripped down twice from ethyl acetate. The product is then placed on the hi-vacuum pump for 2 hr. This affords 84 mg of a white solid (83% yield). $^{13}C$—NMR ($d_6$-Acetone) 138.2, 129.1, 128.3, 94.2, 74.9, 74.4, 74.0, 73.0, 67.2, 66.7, 66.5, 66.3, 65.1, 64.7, 60.3, 57.5, 57.4, 55.8, 52.5, 40.2, 31.5, 23.4, 21.2 and 11.9 δ.

B. In 5 ml of methanol are dissolved 80 mg (0.12 mmol) of N,N'-dicarbobenzyloxy-3'-(n-propylaminomethyl)dihydrospectinomycin, reaction product of part A. To this solution are added 93 mg of palladium black and 46 μl of formic acid. The reaction is stirred for 30 min, filtered, and the solvent removed in vacuo to afford 73 mg of a white solid. The product is taken up in 1 ml of water and 4 ml of 0.1 N HCl are added. The sample is frozen and lyophilized to afford 71 mg of a white solid (100%): $^{13}C$—NMR ($D_2O$) 92.6, 91.7, 71.9, 69.8, 67.0, 66.1, 65.9, 61.5, 60.3, 58.0, 51.4, 50.5, 38.2, 31.2, 19.9, 18.8 and 10.4 δ.

Exact mass calc'd for $C_{29}H_{64}M_3O_7Si_4$ (M+ —$CH_3$): 678.3821;
Found: 678.3791.

Example 29

3'-R-3'-[N-(3-dimethylaminopropyl)aminomethyl]dihydrospectinomycin tetrahydrochloride

A. The epoxide (600 mg, 0.98 mmol, Example 16, part A) is dissolved in 5 ml of 3-dimethylaminopropylamine and heated at 60° for 71 hr. The excess is removed in vacuo and the residue is dissolved in 75 ml of EtOAc which is extracted with 2 × 5 ml of $H_2O$ and 25 ml of brine, dried with $MgSO_4$ and concentrated to give 600 mg of pale yellow solid. The product is chromatographed on 30 g of 230—400 mesh silica gel packed with 5% $MeOH/CHCl_3$ and eluted with 500 ml of 5% $MeOH/CHCl_3$ with 0.5% $NH_4OH$, 1 L of 10% $MeOH/CHCl_3$ with 1% $NH_4OH$ and the rest 20% $MeOH/CHCl_3$ with 1% $NH_4OH$ collecting 30 ml fractions. Pooling of fractions 29-56 yields 235 g (0.33 mmol, 33%) of product as an off white solid: Rf = 0.22 (silica gel, 20% $MeOH/CHCl_3$ with 1% $NH_4OH$); $^{13}C$—NMR ($CD_3COCD_3$) δ 157, 138.2, 129.1, 128.4, 96.9, 93.8, 74.6, 74.2, 71.8, 67.1, 66.4, 65.2, 60.5, 58.1, 57.6, 54.4, 48.1, 45.4, 44.2, 31.7, 27.3, 21.5;

MS exact mass for M+ —$CH_3$ on tetrakistrimethylsilyl derivative, $C_{47}H_{81}N_4O_{11}Si_4$.
Calc'd: 989.4979;
Found: 989.4968.
MS 1004, 989, 493, 449, 359, 305, 273, 187, 115, 91, 58.

B. To a solution of 230 mg (0.32 mmol) of the reaction product of part A in 5 ml of methanol are added 150 mg of palladium black and 0.12 ml (3.2 mmol) of formic acid. The mixture is stirred 3 hr at room temperature and filtered rinsing the catalyst with methanol. The solvent is removed in vacuo and the residue is dissolved in $H_2O$ and treated with 1.4 ml (1.4 mmol) of 1N HCl. Lyophilization yields 200 mg (0.33 mmol), 100% of product as an off white solid: $R_f$ = 0.77 (silica gel, $CHCl_3/MeOH/NH_4OH$, 3/4/2); $^{13}C$—NMR ($D_2O$, $CH_3CN$ internal reference) δ 94.1, 93.2, 73.3, 70.5, 68.2, 66.9, 66.2, 62.4, 60.6 59.3, 55.2, 51.4, 46.4, 43.9, 40.8, 31.9, 31.5, 21.7, 21.0; MS (hexakistrimethylsilyl derivative) 880 (M+), 865, 791, 273, 243, 230, 217, 187, 145, 116, 73, 58;

Exact mass for M+ —$CH_3$, $C_{37}H_{85}N_4O_7Si_6$
Calc'd: 865.5034;
Found: 865.5037.

Example 30

N,N'-dicarbobenzyloxy-3'-(R)-3'-(N-butylaminomethyl)-3'-dihydrospectinomycin (Formula $XIV_b$)

Refer to Chart C.

The formula LII α-epoxide of Example 16, part A (40 mg, 0.065 mmol) is dissolved in 2.0 ml of n-butylamine and warmed to 60° with protection from atmospheric moisture for 18 hr. The solvent is removed in vacuo, 5 ml of ethyl acetate are added and the solvent is removed again. The residue is purified by chromatography on a 20 × 20 cm 250 μm silica gel preparative TLC plate with 254 mm phosphor, eluted with 10% methanol/$CHCl_3$. The band with Rf 0.6—7.5 (visualized by short wave UV light) is collected and the product is washed off of the silica gel with ethyl acetate. Concentration in vacuo gives 28 mg (0.040 mmol, 63%) of title product. $^{13}C$—NMR ($d_6$-Acetone) δ 157, 138.2, 129.1, 128.3, 128.1, 97.1, 93.8, 74.6, 74 (broad), 71.7, 67.1, 66.4, 65.2, 61.0, 60.2, 57.6, 54.4, 49.3, 44.5, 32.1, 31.6, 21.5, 20.8, 14.2; Rf = 0.5 (silica gel, 10% methanol/$CHCl_3$); MS (for tetratrimethylsilyl derivative) m/z 975, 960, 800, 709, 675, 620, 545, 493, 449, 359, 305, 273, 91.

Exact mass for M+ —$CH_3$ calc'd for $C_{46}H_{78}N_3O_{11}Si_4$: 960.4713;
Found: 960.4703.

Example 31

3-(R)-3'-(N-butylaminomethyl)-3'-dihydrospectinomycin trihydrochloride

Refer to Chart C.

The Example 30 product is dissolved in 0.5 ml of methanol and 30 mg of palladium black are added followed by 22 μl (0.58 mmol) of 95% formic acid. The mixture is stirred 1 hr at room temperature and

filtered, rinsing the catalyst with three portions of methanol. The solvent is removed in vacuo and the residue is dissolved in 4 ml of water. The pH is adjusted to 1.5 with 1N HCl and the solution was lyophilized to afford 21.5 mg of title product as a white solid: $^{13}C$—NMR ($D_2O$, $CH_3CN$ reference) δ 94.1, 93.3, 73.3, 70.5, 68.3, 66.9, 66.2, 62.5, 60.6, 59.4, 51.0, 49.5, 40.9, 31.9, 31.5, 27.9, 21.0, 20.1, 13.9; Rf = 0.85 (silica gel, $CHCl_3$/methanol/$NH_4OH$, 3:4:2); MS (for tetrakistrimethylsilyl derivative) m/z 707, 692, 621, 349, 273, 171, 158, 145, 86, 73.

Exact mass for M+ —$CH_3$ calc'd for $C_{30}H_{66}N_3O_7Si_4$: 692.3978;
Found: 692.3967.

Example 32

N,N'-dibenzyloxycarbonyl-3'-(S)-spectinomycin cyanohydrin (Formula $XVII_b$)
Refer to Chart D.

In 25 ml of methanol are combined 5.0 gm (8.33 mmol) of N,N'-dibenzyloxycarbonylspectinomycin and 0.76 ml (8.33 mmol) of acetone cyanohydrin. To this solution are then added 100 mg (0.72 mmol) of $K_2CO_3$. The reaction is stirred from 4.5 hr and then concentrated in vacuo. The residue is taken up in 40 ml of ethyl acetate, washed once with 50 ml brine and dried over $Na_2SO_4$. The solution is then filtered and concentrated in vacuo to afford 5.0 gm of a white solid. The product is taken up in $CHCl_3$ and chromatographed on 200 gm of silica, slurry packed in $CHCl_3$. The column is eluted as follows: 1 L 1% methanol/$CHCl_3$; 1 L 2% methanol/$CHCl_3$; 2 L 4% methanol/$CHCl_3$; 2 L 5% methanol/$CHCl_3$. In elution volume 3.3 L to 6.0 L is found 3.92 g of the title compound (75% yield): $^{13}C$—NMR ($d_4$-methanol) 138.1, 138.0, 129.4, 129.2, 128.8, 128.4, 94.4, 91.1, 74.8, 74.6, 74.5, 73.3, 68.2, 66.8, 66.4, 66.2, 60.9, 57.8, 42.1, 31.7 and 20.6 ppm; $[\alpha]_D$ +4° (C, 0.8935, $CHCl_3$).

Mass spec m/e 816, 801, 745, 629, 493, 449, 359, 305, 270, 170, 144, 91 and 73.

Example 33

3'-(S)-Spectinomycin cyanohydrin diformate
Refer to Chart D.

In 5 ml of methanol are dissolved 200 mg (0.32 mmol) of N,N-dicarbobenzyloxyspectinomycin cyanohydrin. To this solution are added 200 mg of Pd black followed by 120 µl (3.2 mmol) of formic acid. The reaction is stirred for 0.5 hr and is filtered. Removal of the solvent in vacuo afforded 170 mg of title product, a white solid: $^{13}C$—NMR ($D_2O$) 93.5, 91.3, 73.0, 71.3, 68.2, 67.5, 67.4, 67.2, 62.7, 59.6, 41.3, 33.6, 32.0 and 20.6.

Example 34

N,N'-dibenzyloxycarbonyl-3'-(S)-3'-(aminomethyl)dihydrospectinomycin (Formula $XIX_b$)
Refer to Chart D.

In 10 ml of acetic acid are dissolved 3.33 g (5.31 mmol) of N,N'-dibenzyloxycarbonyl-3'-(S)-spectinomycin cyanohydrin (Example 32). The solution is added to a Paar bottle containing 5.0 g of wet Raney nickel. The solution is then hydrogenated for 3.5 hr at an initial pressure of 103 kPa (15 psi). The solution is then filtered through magnesium silicate and the acetic acid is removed in vacuo. The residue is partitioned between water and ethyl acetate. The water is separated and combined with 2 × 100 ml 1N HCl washes of the ethyl acetate. The aqueous washes are made alkaline with 1N NaOH (pH = 10) and extracted with ethyl acetate (2 × 100 ml). The combined organic extracts are then washed with 100 ml of brine and are dried over $MgSO_4$. Afer filtering, removal of the solvent in vacuo leaves 1.323 g (40% yield) of the title compound: $^{13}C$—NMR ($d_4$-methanol) 158.9, 158.4, 138.1, 129.5, 129.0, 128.7, 94.8, 93.7, 74.7, 73.9, 73.3, 68.7, 68.3, 67.7, 66.8, 65.7, 61.2, 57.9, 45.8, 39.7, 31.8 and 21.2 ppm.

Example 35

N,N'-dibenzyloxycarbonyl-3'-(R)-spectinomycin cyanohydrin (Formula $XVIII_b$)
Refer to Chart E.

In 1 ml of methanol are combined 1.0 g (1.66 mmol) of N,N'-dibenzyloxycarbonylspectinomycin and 0.152 ml (1.66 mmol) of acetone cyanohydrin. To this solution is added 0.5 g of Amberlite IR—45 C.P. resin, followed by 1 ml of saturated aqueous $NH_4Cl$. The reaction is stirred for 24 hr at room temperature. The reaction is then filtered and the methanol removed in vacuo, the residue is partitioned between ethyl acetate and water. The ethyl acetate is separated and combined with the ethyl acetate extract of the aqueous phase. The combined extracts are washed with brine and dried over $MgSO_4$. After filtering, the solvent is removed in vacuo to afford 1.229 g of a white solid. The material is taken up in $CHCl_3$ and chromatographed on 35 g of silica, slurry packed in $CHCl_3$. The column is eluted as follows: 200 ml 1% methanol/$CHCl_3$; 1 L 2% methanol/$CHCl_3$ and 1 L 4% methanol/$CHCl_3$. Elution volume 600—850 ml contains 364 mg of N,N'-dibenzyloxycarbonylspectinomycin. Elution volume 1100—1200 ml contains 131 mg of the title compound while 207 mg of N,N'-dibenzyloxycarbonyl-3'-(S)-spectinomycin cyanohydrin is found in elution volume 1450 ml—2100 ml. The yield of the desired cyanohydrin is 20%: $^{13}C$—NMR ($d_6$-Acetone) 156.9, 137.8, 129.0, 128.3, 120.1, 95.2, 91.5, 73.3, 74.8, 74.5, 74.1, 73.9, 67.8, 67.3, 66.2, 60.7, 60.6, 60.4, 57.6, 57.5, 57.3, 42.0, 31.7 and 20.6 ppm.

Example 36

N,N'-dibenzyloxycarbonyl-3'-(R)-3'-(aminomethyl)dihydrospectinomycin (Formula $XX_b$)

Refer to Chart E.

In 1 ml of acetic acid are dissolved 130 mg (0.21 mmol) of N,N'-dibenzyloxycarbonyl-3'-(R)-spectinomycin cyanohydrin (Example 35). This solution is added to a Paar bottle containing 0.5 gm of wet Raney-Nickel. The solution is hydrogenated at 103 kPa (15 psi) for 2 hr. The solution is filtered through magnesium silicate and rinsed with methanol. The filtrate is concentrated in vacuo and the residue partitioned between diethyl ether and water. The aqueous phase is separated and made alkaline with the addition of concentrated $NH_4OH$ (pH-9). The basic solution is extracted with ethyl acetate (2 × 20 ml). The combined extracts are washed with 20 ml of brine and dried over $MgSO_4$. After filtering, removal of the solvent leaves 73 mg of the title compound (56.6% yield).

Example 37

N,N'-dibenzyloxycarbonyl-3'-(R)-3'-(aminomethyl)dihydrospectinomycin (Formula $XX_b$)

In 25 ml of methanol are dissolved 10.0 g (16.6 mmol) N,N'-dicarbobenzyloxy-spectinomycin and 7.6 ml (83.2 mmol) of acetone cyanohydrin. To this solution are added 5.0 g of IR—45 C.P. resin. The mixture is stirred for 3.5 hr, filtered and concentrated in vacuo. The residue is taken up in ethyl acetate and washed with water (2 × 50 ml). The combined washes are backwashed with ethyl acetate (1 × 20 ml). The combined organics are washed with 50 ml of brine and dried over $MgSO_4$. After filtering, removal of the solvent in vacuo affords 11.34 g of a white solid. By TLC the products consist of unreacted material and both formula LXVII and LXVIII cyanohydrin epimers. The mixture is dissolved in 50 ml of acetic acid and the solution is added to a Paar bottle containing 17 g of wet Raney-Nickel. The solution is hydrogenated at 15 psi for 1 hr, and filtered through Magensol. The filtrate is concentrated in vacuo and the residue partitioned between diethyl ether and water. Enough ethyl acetate is added to solubilize everything. The aqueous phase is separated and made alkaline with concentrated $NH_4OH$. The basic solution is extracted with ethyl acetate. An emulsion forms which is broken up by filtering through diatomaceous earth. The ethyl acetate is separated and combined with a 100 ml ethyl acetate extract of the aqueous phase. The combined extracts are washed with brine, dried over $MgSO_4$, and filtered. Removal of the solvent affords 3.49 g of a blue solid. The product is taken up in $CHCl_3$ and chromatographed on 100 g of silica, slurry packed in $CHCl_3$. The column is eluted as follows, with 1% aqueous $NH_4OH$ added to the mobile phase: 200 ml 1% methanol/$CHCl_3$; 200 ml 2% methanol/$CHCl_3$; 200 ml 3% methanol/$CHCl_3$; 200 ml 4% methanol/$CHCl_3$; 3 L 5% methanol/$CHCl_3$; 1 L 10% methanol/$CHCl_3$. In elution volume 2.5 l to 4.2 l there are obtained 1.173 g of the title compound as a white solid (11.2% yield, based on starting material: $^{13}C$—NMR ($d_4$-methanol) 158.23, 138.0, 129.4, 128.8, 128.6, 96.3, 94.3, 74.6, 73.8, 68.1, 66.7, 65.5, 60.9, 60.8, 58.0, 45.6, 41.4, 31.8 and 21.5 ppm.

Example 38

N,N'-dibenzyloxycarbonyl-3'-(R)-3'-(N-hexylaminomethyl)dihydrospectinomycin and N,N'-dicarbobenzyloxy-3'-(N,N'-dihexylamino-methyl)dihydrospectinomycin (Formula $XXII_b$)

In 10 ml of methanol are combined 500 mg (0.79 mmol) of N,N'-dibenzyloxycarbonyl-3'-(R)-3'-(aminomethyl)dihydrospectinomycin, 0.95 ml (7.9 mmol) of n-hexanal and 16.5 mg (0.26 mmol) of $NaCNBH_3$. A trace of methyl orange indicator is added and the pH is adjusted to the indicator's end point using 1N HCl/methanol. The reaction is stirred for 2 hr at room temperature, during which time an additional 33 mg (0.52 mmol) of $NaCNBH_3$ is added. The solvent is then removed in vacuo and the residue partitioned between water and ethyl acetate.

The ethyl acetate is separated and combined with a 20 ml ethyl acetate extract of the aqueous phase. The combined extracts are washed with 50 ml of brine and dried over $MgSO_4$. After filtering, removal of the solvent affords an oil which is immediately taken up in $CHCl_3$ and chromatographed on 35 g of silica, slurry packed in $CHCl_3$. The column is eluted as follows: 100 ml $CHCl_3$; 200 ml 1% methanol/$CHCl_3$; 200 ml 2% methanol/$CHCl_3$; 200 ml 3% methanol/$CHCl_3$; and 1 liter of 5% methanol/$CHCl_3$. In elution volume 400—900 ml there are obtained 310 mg (49% yield) of N,N'-dibenzyloxycarbonyl-3'-(R)-3'-(N,N-dihexylaminomethyl)dihydrospectinomycin as a white solid: $^{13}C$—NMR ($d_6$-Acetone) 138.2, 129.1, 128.7, 128.4, 128.2, 97.2, 93.8, 74.7, 74.4, 72.2. 67.2, 66.5, 65.4, 65.2, 61.0, 60.4, 60.3, 59.7, 57.7, 57.5, 55.9, 44.6, 32.4, 32.2, 32.0, 31.8, 31.5, 27.6, 26.9, 23.2, 21.5 and 14.3 ppm

| | |
|---|---|
| Exact mass calc'd for $C_{54}H_{94}N_3O_{11}Si_4$ (tetra-TMS-$CH_3$): | 1072.5965; |
| Found: | 1072.5968. |

Elution volume 950—1400 ml contains 109 mg (19.3%) of N,N'-dibenzyloxycarbonyl-3'-(R)-3'-(N-hexylaminomethyl)-dihydrospectinomycin as a white solid: $^{13}C$—NMR ($d_6$-Acetone) 138.1, 129.1, 128.3, 128.1, 97.0, 93.8, 74.5, 71.7, 67.2, 66.4, 65.2, 57.6, 54.3, 49.7, 44.4, 32.2, 31.6, 29.9, 27.4, 23.1, 21.5 and 14.2 ppm.

| | |
|---|---|
| Exact mass calc'd for $C_{49}H_{85}N_3O_{11}Si_4$ (tetra TMS): | 1003.5261; |
| Found: | 1003.5278. |

Example 39

N,N'-dibenzyloxycarbonyl-3'-(R)-3'-(N-isobutylaminomethyl)dihydrospectinomycin and 3'-(N,N-diisobutylaminomethyl)dihydrospectinomycin (Formula $XXII_b$)

Refer to Chart E.

To a solution of 500 mg (0.79 mmol) of N,N'-dibenzyloxycarbonyl-3'-(R)-3'-aminomethyl)-dihydrospectinomycin in 10 ml of methyl alcohol are added 0.72 ml (7.9 mmol) of isobutyraldehyde and a trace of methyl orange indicator. The pH is adjusted to the indicator's end point with methanonlic HCl and 16.5 mg (0.26 mmol) of sodium cyanoborohydride in 1 ml of methyl alcohol is added. The reaction is stirred at room temperature and the pH is maintained near the endpoint of methyl orange by adding additional methanolic HCl. The reaction is easily followed by TLC using Analtech Silica plates and eluting with 10% $MeOH/CHCl_3$/ 1% $NH_4OH$ as a solvent. When the reaction is complete, the solvent is removed in vacuo and the residue is partitioned between $H_2O$ and EtOAc. The aqueous phase is made alkaline with the addition of concentrated $NH_4OH$ and the EtOAc is separated and combined with an additional EtOAc extract. The combined extracts are washed with brine and dried over $MgSO_4$. The solvent is then removed in vacuo to afford the crude product. The product is taken up in $CHCl_3$ and chromatographed on silica, slurry packed in $CHCl_3$. The column is eluted with a $MeOH/CHCl_3$ gradient (1%—5%), and 50 ml fractions are taken. The fractions are analyzed by TLC and pure fractions are pooled and concentrated in vacuo to afford 216 mg (39.8%) of N,N'-dibenzyloxycarbonyl-3'-(R)-3'-(N-isobutylaminomethyl)dihydrospectinomycin: $^{13}C$—NMR ($d_6$-acetone): 138.1, 129.1, 128.6, 128.3, 128.1, 97.1, 93.8, 74.5, 71.7, 67.2, 66.4, 65.2, 57.8, 54.6, 44.4, 31.8, 31.6, 28.4, 21.5, 20.9 and 20.8 ppm.

| | |
|---|---|
| Exact mass calc'd for $C_{47}H_{81}N_3O_{11}Si_4$ (tetra TMS): | 975.4948; |
| Found: | 975.4943. |

The same procedure, using 631 mg (1.0 mmol) of the amine, 1.82 ml (200 mmol) of aldehyde and 188 mg (3.0 mmol) of sodium cyanoborohydride afforded 171 mg (23%) of N,N'-dibenzyloxycarbonyl-3'-(R)-3'-(N,N-diisobutylaminomethyl)-dihydrospectinomycin: $^{13}C$—NMR ($d_6$-acetone): 138.1, 129.1, 128.3, 128.1, 97.0, 93.5, 74.6, 73.1, 67.3, 67.2, 66.4, 65.2, 61.1, 57.5, 44.1, 31.4, 26.8, 21.7 and 21.4 ppm;

| | |
|---|---|
| Exact mass calc'd for $C_{48}H_{81}N_3O_{11}Si_4$ (tri-TMS): | 959.5179; |
| Found: | 959.5134. |

Example 40

N,N'-dibenzyloxycarbonyl-3'-(R)-3'-(N-cyclohexylmethylaminomethyl)dihydrospectinomycin and N,N'-dibenzyloxycarbonyl-3'-(R)-3'-[N,N-di(cyclohexylmethyl)aminomethyl]dihydrospectinomycin (Formula $XXII_b$)

Alkylation of 2.4 g (3.9 mmol) of N,N'-dibenzyloxycarbonyl-3'-(R)-3'-(aminomethyl)dihydrospectinomycin using 2.11 ml (19.5 mmol) of cyclohexane carboxaldehye and 180 mg (2.8 mmol) of sodium cyanoborohydride following the procedure in Example 39 afforded 982 mg (31%) of N,N'-dibenzyloxycarbonyl-3'-(R)-3'-(N-cyclohexylmethylaminomethyl)dihydrospectinomycin: $^{13}C$—NMR ($d_6$-Acetone): 138.1, 129.1, 128.3, 97.0, 93.7, 74.5, 71.7, 67.2, 66.5, 65.2, 57.6, 56.5, 54.6, 44.5, 38.0, 31.9, 31.6, 27.2, 26.5 and 21.5 ppm;

| | |
|---|---|
| Exact mass calc'd for $C_{50}H_{85}N_3O_{11}Si_4$ (tetra TMS): | 1015.5261; |
| Found: | 1015.5240. |

Alkylation of 634 mg (1.0 mmol) of the amine using 1.08 ml (10 mmol) of aldehyde and 62.8 mg (1.0 mmol) of sodium cyanoborohydride gave 173 mg (21%) of N,N'-dibenzyloxycarbonyl-3'-(R)-3'-[N,N-di-(cyclohexylmethyl)-aminomethyl]-dihydrospectinomycin: $^{13}C$—NMR ($d_6$-Acetone): 129.1, 128.4, 128.2, 97.3, 93.5, 74.7, 72.9, 67.4, 67.2, 66.5, 65.1, 61.2, 57.7, 57.6, 57.4, 44.6, 36.5, 32.6, 32.4, 31.7, 27.2, 26.7 and 21.4 ppm;

| | |
|---|---|
| Exact mass calc'd for $C_{53}H_{86}N_3O_{11}Si_3$ (tri TMS—$CH_3$): | 1024.5570; |
| Found: | 1024.5526. |

Example 41

Deprotection of the N,N'-dibenzyloxycarbonyl-3'-(R)-3'-mono and di-alkylaminomethyl-dihydrospectinomycin of Examples 38—40

The protected products of Examples 38, 39 or 40 is dissolved in methanol and an equivalent weight of Pd black is added. To this mixture are added 20 equivalents of formic acid. The reaction is stirred for 45 min, filtered and concentrated to dryness. The residue is taken up in water and 3.5 equivalents of 1N HCl are added. The solution is frozen and lyophilized to afford the following final products:

(a) 3'-(R)-3'-(N-hexylaminomethyl)dihydrospectinomycin trihydrochloride:

Deprotection of 87 mg (0.12 mmol) affords 58 mg (87% yield) of the title compound as a while solid: $^{13}C$—NMR ($D_2O$) 94.1, 93.3, 73.2, 70.5, 68.3, 66.9, 66.2, 62.5, 60.6, 59.3, 51.0, 49.8, 40.9, 31.9, 31.5, 31.4, 26.3, 25.8, 22.7, 21.0 and 14.3 ppm.

| | |
|---|---|
| Exact mass calc'd for $C_{39}H_{89}N_3O_7Si_6$ (hexa-TMS): | 879.5316; |
| Found: | 879.5303. |

(b) 3'-(R)-3'-(N-isobutylaminomethyl)dihydrospectinomycin trihydrochloride:

Deprotection of 216 mg (0.29 mmol) affords 152 mg (90% yield) of the title compound as a white solid: $^{13}C$—NMR ($D_2O$) 93.9, 93.3, 73.0, 70.3, 68.1, 66.8, 66.1, 62.3, 60.5, 59.2, 56.3, 51.3, 41.2, 31.8, 31.4, 25.5, 21.0, 19.9 and 19.7 ppm;

Exact mass calc'd for $C_{37}H_{85}N_3O_7Si_6$ (hexa TMS): 851.5003;
Found: 851.5020.

(c) 3'-(R)-3'-(N-cyclohexylmethylaminomethyl)dihydrospectinomycin trihydrochloride:

Deprotection of 928 mg (1.12 mmol) afford 640 mg of the title compound as a white solid (86% yield): $^{13}C$—NMR ($D_2O$) 94.2, 93.5, 73.2, 70.6, 68.3, 66.9, 66.3, 62.5, 60.6, 59.4, 55.3, 51.5, 41.3, 34.6, 31.9, 31.5, 30.7, 26.5, 25.9 and 21.0 ppm;

Exact mass calc'd for $C_{40}H_{89}N_3O_7Si_6$ (hexa TMS): 891.5316;
Found: 891.5324.

(d) 3'-(R)-3'-(N,N-dihexylaminomethyl)dihydrospectinomycin trihydrochloride:

Deprotection of 220 mg (0.28 mmol) affords 157 mg (88% yield) of the title compound as a white solid: $^{13}C$—NMR ($D_2O$) 94.1, 93.3, 70.5, 68.3, 68.1, 66.7, 66.6, 66.5, 66.3, 62.4, 60.5, 59.3, 56.9, 56.7, 31.9, 31.5, 26.3, 23.7, 22.8, 21.0 and 14.4 ppm.

Exact mass calc'd for $C_{42}H_{93}N_3O_7Si_6$ (hexa TMS): 891.5860;
Found: 891.5853.

(e) 3'-(R)-3'-(N,N-diisobutylaminomethyl)dihydrospectinomycin trihydrochloride:

Deprotection of 153 mg (0.20 mmol) affords 96 mg of the title compound (82% yield): $^{13}C$—NMR ($D_2O$) 94.3, 93.9, 73.3, 70.6, 68.2, 66.7, 66.6, 65.7, 62.6, 60.7, 59.6, 43.3, 31.9, 31.8, 31.6, 25.1, 24.8, 21.1, 21.0, 20.6, 20.4, 19.8 and 19.6 ppm

Exact mass calc'd for $C_{38}H_{85}N_3O_7Si_5$ (penta-TMS): 835.5234;
Found: 835.5241.

(f) 3'-(R)-3'-(N,N-di[cyclohexylmethyl]aminomethyl)dihydrospectinomycin trihydrochloride:

Deprotection of 149 mg (0.18 mmol) affords 124 mg of the title compound as a white solid (100% yield): $^{13}C$—NMR ($D_2O$) 94.3, 93.8, 73.2, 70.5, 68.2, 66.9, 66.5, 65.2, 64.5, 62.6, 60.7, 59.5, 43.2, 34.1, 33.7, 31.9, 31.8, 31.3, 30.9, 30.7, 26.5, 25.9 and 21.1 ppm;

Exact mass calc'd for $C_{41}H_{85}N_3O_7Si_4$ (tetra TMS): 843.5465;
Found: 843.5459.

## Example 42

N,N'-dibenzyloxycarbonyl-3'-(R)-3'-(N-cycloalkylaminomethyl)dihydrospectinomycins (Formula $XXII_b$)
Refer to Chart E.

In 3 ml of methanol are combined 1 mmol of N,N'-dibenzyloxycarbonyl-3'-(R)-3'-(aminomethyl)dihydrospectinomycin (Example 36) and 10 mmol of cycloalkanone. To this solution is added 0.33 mmol of sodium cyanoborohydride. The pH is adjusted to 6 with methanolic hydrogen chloride. The reaction is followed by TLC on silica eluting with 90:10:1 chloroform/methanol/concentrated aqueous ammonia. When the reaction is complete, the solvent is removed in vacuo and the residue partitioned between ethyl acetate and water, made alkaline with concentrated aqueous ammonia. The ethyl acetate is separated, combined with a second extract, washed with brine and dried over magnesium sulfate. After filtering, the solvent is removed in vacuo and the residue chromatographed on 40 g of silica, slurry packed in chloroform. The column is eluted with a methanol chloroform gradient and 45 ml fractions are collected. The desired product is found by TLC analysis. The pure fractions are pooled and concentrated to afford the following final products:

(a) N,N'-dibenzyloxycarbonyl-3'-(R)-3'-(N-cyclopentylaminomethyl)dihydrospectinomycin (Formula LXVI: $R_{63}$ is cyclopentyl and $R_{64}$ is hydrogen).

The title compound is obtained in a 47% yield, 328 mg of a white solid were obtained: $^{13}C$—NMR ($d_6$-acetone) 157.0, 138.2, 129.1, 128.3, 128.1, 97.2, 93.8, 74.6, 71.7, 67.1, 66.0, 65.2, 60.9, 60.2, 59.6, 57.7, 57.6, 53.0, 44.6, 33.3, 32.5, 31.6, 24.4, 24.3 and 21.5 ppm;

Exact mass calc'd for $C_{48}H_{81}N_3O_{11}Si_4$ (tetra TMS): 987.4948;
Found: 987.4924.

(b) N,N'-dibenzyloxycarbonyl-3'-(R)-3'-(N-cyclohexylaminomethyl)dihydrospectinomycin (Formula LXVI: $R_{63}$ is cyclohexyl and $R_{64}$ is hydrogen.)

Chromatography affords 240 mg (34% yield) of the title compound as a white solid: $^{13}C$—NMR ($d_6$-acetone) 157.0, 138.2, 129.1, 128.3, 128.1, 97.2, 93.8, 74.6, 71.6, 67.1, 67.0, 66.4, 65.2, 60.9, 60.2, 57.6, 57.5, 56.6, 51.4, 44.5, 33.4, 33.3, 31.6, 26.6, 25.4 and 21.5 ppm

Exact mass calc'd for $C_{49}H_{83}N_3O_{11}Si_4$ (tetra TMS): 1001.5104;
Found: 1001.5132.

(c) N,N'-dibenzyloxycarbonyl-3'-(R)-3'(N-cycloheptylaminomethyl)dihydrospectinomycin (Formula LXVI: $R_{63}$ is cycloheptyl and $R_{64}$ is hydrogen.)

Chromatography affords 178 mg (24.5%) of the title compound as a white solid: $^{13}C$—NMR ($d_6$-acetone) 157.0, 138.0, 129.0, 128.2, 128.0, 97.0, 93.6, 74.5, 71.5, 67.1, 66.4, 65.2, 60.6, 60.0, 58.7, 57.5, 51.7, 44.5, 34.7, 34.6, 31.6, 28.8, 24.5 and 21.4 ppm;

Exact mass calc'd for $C_{50}H_{85}N_3O_{11}Si_4$: 1015.5261;
Found: 1015.5282.

Example 43

Preparation of 3'-(R)-3'-(N-cycloalkylaminomethyl)dihydrospectinomycin trihydrochlorides

Refer to Chart E.

The N,N'-dibenzyloxycarbonyl-3'-(R)-3'-(cycloalkylaminomethyl)dihydrospectinomycin (Example 42) and an equal weight of palladium black are combined in 10 ml of methanol. To the mixture are added 10 equivalents of formic acid. After stirring for 20 min, the reaction mixture is filtered and the solvent is removed in vacuo. The solid residue is taken up in water and treated with 3.5 equivalents of 1N hydrochloric acid. The solution is frozen and lyophilized to afford the following final products:

(a) 3'-(R)-3'-(N-cyclopentylaminomethyl)dihydrospectinomycin trihydrochloride

Deprotection of 320 mg (0.45 mmol) affords 244 mg (100%) of the title compound as a white solid: $^{13}C$—NMR ($D_2O$) 94.2, 93.3, 73.4, 70.6, 68.4, 67.0, 66.3, 62.6, 61.8, 60.7, 59.4, 50.4, 40.7, 31.9, 31.5, 30.1, 24.7 and 21.0 ppm;

Exact mass calc'd for $C_{39}H_{87}N_3O_7Si_6$ (hexa-TMS): 877.5159;
Found: 877.5143.

(b) 3'-(R)-3'-(N-cyclohexylaminomethyl)dihydrospectinomycin trihydrochloride

Deprotection of 240 mg (0.33 mmol) affords 154 mg (84.1% yield) of the title compound as a white solid: $^{13}C$—NMR ($D_2O$) 94.1, 93.3, 73.3, 70.6, 68.3, 66.9, 66.3, 62.5, 60.6, 59.8, 59.4, 48.1, 40.8, 31.9, 31.5, 29.5, 29.3, 25.4, 25.0 and 21.0 ppm.

Exact mass calc'd for $C_{39}H_{87}N_3O_7Si_6$ (hexa TMS): 877.5159;
Found: 877.5143.

(c) 3'-(R)-3'-(N-cycloheptylaminomethyl)dihydrospectinomycin trihydrochloride

Deprotection of 160 mg (0.22 mmol) affords 120 mg (96%) of the title compound as a white solid: $^{13}C$—NMR ($D_2O$) 94.1, 93.3, 73.3, 70.5, 68.3, 66.9, 66.3, 62.5, 61.7, 60.6, 59.3, 48.1, 40.9, 31.9, 31.5, 31.1, 27.9, 24.6 and 21.0 ppm;

Exact mass calc'd for $C_{40}H_{89}N_3O_7Si_6$ (hexa TMS): 891.5316;
Found: 891.5350.

Example 44

N,N'-dibenzyloxycarbonyl-3'-(R)-3'-(N-acetylaminomethyl)dihydrospectinomycin (Formula $XXIV_b$: R methyl).

Refer to Chart E.

To a solution of 631 mg (1.0 mmol) of N,N'-dibenzyloxycarbonyl-3'-(R)-3'-aminomethyldihydrospectinomycin in 4 ml of methanol stirred at 0° under a nitrogen atmosphere are added 0.15 ml (1.1 mmol) of triethylamine and 0.10 ml (1.1 mmol) of acetic anhydride. The mixture is stirred 1.15 hr at 0°, and then concentrated in vacuo. The residue is dissolved in 35 ml of ethyl acetate and washed twice with 5 ml of water, once with 20 ml of brine and dried over $MgSO_4$. Removal of solvent in vacuo affords 600 mg of white solid. The product is purified by chromatography on 40 g of 230—400 mesh silica gel packed with 1% methanol/chloroform and eluted with 1 l of 1% methanol/chloroform, 2 l of 2% methanol/chloroform and finally 3% methanol/chloroform, taking 45 ml fractions. Fractions 71—98 are pooled and concentrated to give 212 mg (0.32 mmol, 32%) of title product as a white solid: $^{13}C$—NMR ($d_6$-acetone) δ 171.7, 157.0, 138.2, 129.1, 128.4, 95.0, 93.3, 75.8, 74.5, 67.7, 67.4, 70.0, 66.6, 60.0, 57.0, 42.2, 39.6, 31.8, 22.9, and 21.5; MS exact mass for pentatrimethylsilyl derivative $C_{48}H_{83}N_3O_{12}Si_5$. Calc'd: 1033.4823. Found: 1033.4807.

Example 45

3'-(R)-3'-(acetylaminomethyl)dihydrospectinomycin dihydrochloride.

Refer to Chart E.

To a solution of 205 mg (0.30 mmol) of the title product of Example 44 in 5 ml of methanol are added 200 mg of palladium black and 0.11 ml (3.0 mmol) of formic acid. The mixture is stirred 30 min at room temperature, filtered and concentrated in vacuo. The residue is dissolved in 5 ml of $H_2O$, treated with 0.6 ml (0.6 mmol) of 1.0 N HCl and lyophilized to afford 200 mg of white solid: $^{13}C$—NMR ($D_2O$, $CH_3CN$ internal reference) δ 175.5, 94.3, 93.4, 76.1, 70.5, 68.7, 66.9, 65.9, 62.4, 60.5, 59.2, 42.6, 39.1, 31.9, 31.3, 22.8, and 21.0; MS exact mass for hexatrimethylsilyl derivative $C_{35}H_{79}N_3O_8Si_6$. Calc'd: 837.4483. Found: 837.4472.

Example 46

3'-(R)-3'-[N-(pyroll-2-ylcarbonyl)aminomethyl]dihydrospectinomycin dihydrochloride.

Refer to Chart E.

A. To a solution of 631 mg (1.0 mmol) of N,N'-dibenzyloxycarbonyl-3'-(R)-3'-aminomethyldihydrospectinomycin (Example 36) in 20 ml of dry tetrahydrofuran stirred at room temperature with a nitrogen atmosphere are added 216 mg (1.05 mmol) of dicyclohexylcarbodiimide, 10 mg (0.08 mmol) of 4-dimethylaminopyridine and 111 mg (1.0 mmol) of pyrolle-2-carboxylic acid. The mixture is stirred 2 hr at room temperature and 4 equivalents of each of the last three reactants are added. After 2 hr stirring, the mixture is concentrated in vacuo, diluted with 200 ml of EtOAc and extracted with 50 ml of 5% $NaHCO_3$. Some insoluble material is removed with the aqueous layer. The EtOAc solution is washed with 5% $NaHCO_3$ and brine and dried with $MgSO_4$. Concentration in vacuo affords 1.43 g of brown gum.

The product is chromatographed on 140 g of 230—400 mesh silica gel packed with 1% methanol/chloroform and eluted with 1 l of 1% methanol/chloroform and the remainder 2% methanol/chloroform collecting 45 ml fractions. Fractions 105—154 are pooled to afford 252 mg (0.35 mmol, 35%) of the product as a white solid: $^{13}C$—NMR ($d_6$-acetone) δ 162.8, 157.0, 138.2, 129.1, 128.4, 126.6, 122.6, 111.0, 110.0, 95.2, 93.4, 76.1, 74.5, 67.4, 66.5, 65.6, 60.0, 57.8, 55.4, 42.5, 40.0, 31.8, and 21.4; MS for pentatrimethylsilyl ether 1084, 1069, 997, 889, 799, 339, 195, 166, 91; exact mass for $C_{51}H_{84}N_4O_{12}Si_5$. Calc'd: 1084.4932. Found, 1084.4927.

B. To a solution of 240 mg (0.33 mmol) of the reaction product of part A in 5 ml of methanol are added 200 mg of palladium black and 0.125 ml (3.3 mmol) of formic acid. The mixture is stirred 30 min at room temperature, filtered and concentrated in vacuo. The residue is dissolved in 5 ml of $H_2O$, treated with 0.66 ml (0.66 mmol) of 1N HCl and lyophilized to afford 174 mg of the title product as a white solid: $^{13}C$—NMR ($D_2O$, $CH_3CN$ internal reference) δ 164.3, 125.4, 124.2, 113.0, 110.8, 94.4, 93.7, 76.6, 70.7, 68.9, 67.1, 66.1, 62.6, 60.6, 59.4, 42.6, 39.3, 32.0, 31.4, and 21.4; MS exact mass for heptatrimethylsilyl ether $C_{41}H_{88}N_4O_8Si_7$. Calc'd: 960.4987. Found, 960.4982.

Example 47

N,N'-dibenzyloxycarbonyl-3'-(S)-3'-(N-ethylaminomethyl)dihydrospectinomycin (formula $XXI_b$).

Refer to Chart D.

In 6 ml of MeOH are combined 500 mg (0.774 mmol) of N,N'-dibenzyloxycarbonyl-3'-(S)-3'-(aminomethyl)dihydrospectinomycin (Example 34), 44 μl (0.774 mmol) of acetaldehyde and a trace of methyl orange indicator in MeOH. To this solution are then added 16.2 mg (0.258 mmol, 0.774 mmol $H^-$) of $NaCNBH_3$ in 1 ml of MeOH. The pH is adjusted with the addition of 1N methanolic HCl until the indicator is just pink. The reaction is then stirred at room temperature and additional 1N HCl/MeOH is added to keep the indicator pink. After 2.5 hours the MeOH is removed and the residue partitioned between $H_2O$ and EtOAc. The aqueous phase is extracted again with EtOAc (2 × 30 ml). The combined organics are washed with brine (1 × 50 ml) and dried over $MgSO_4$. After filtering, the solvent is removed in vacuo to afford 458 mg of a white solid. The product is taken up in $CHCl_3$ and chromatographed on 20 gm of silica gel, slurry packed in $CHCl_3$. The column is eluted as follows: 100 ml, 1% $MeOH/CHCl_3$; 100 ml, 2% $MeOH/CHCl_3$; 100 ml 3% $MeOH/CHCl_3$; 100 ml 5% $MeOH/CHCl_3$; 100 ml 7% $MeOH/CHCl_3$; and 700 ml 10% $MeOH/CHCl_3$. In elution volume 520—720 ml there is contained 306 mg of the title compound as a white solid (60% yield): $^{13}C$—NMR ($d_6$-acetone) δ 138.1, 129.1, 128.3, 94.2, 74.4, 73.1, 67.2, 66.6, 65.1, 57.6, 57.5, 57.3, 55.7, 45.0, 40.3, 31.4, 21.1, and 15.3 ppm; exact mass calc'd for $C_{41}H_{66}N_3O_{11}Si_3$ (tri-OTMS, —$CH_3$) 860.4005, found 860.4008.

Example 48

N,N'-dibenzyloxycarbonyl-3'-(S)-3'-(N-butylaminomethyl)dihydrospectinomycin (Formula $XXI_b$).

Refer to Chart D.

In 10 ml of MeOH are dissolved 507 mg (0.803 mmol) of N,N'-dibenzyloxycarbonyl-3'-(S)-3'-(aminomethyl) dihydrospectinomycin (Example 34). To this solution are added 0.71 ml (8.0 mmol) of n-butyraldehyde and a drop of methyl orange indicator in MeOH. This is followed by the addition of 17 mg of $NaCNBH_3$ in 1 ml of MeOH. The pH is adjusted to 4 with the addition of 1N HCl/MeOH. The pH is maintained at 4 for 0.5 hr while the reaction is stirred. The MeOH is then removed in vacuo and the residue partitioned between EtOAC and $H_2O$. The EtOAC is separated and combined with 1 × 25 ml EtOAC extract of the aqueous phase. The combined extracts are washed with 50 ml of brine and dried over $MgSO_4$. After filtering, removal of the solvent affords 616 mg of a white solid. The product is taken up in $CHCl_3$ and chromatrographed on 30 g of silica gel, slurry packed in $CHCl_3$. The column is eluted as follows: 100 ml 1% $MeOH/CHCl_3$; 100 ml 2% $MeOH/CHCl_3$; 100 ml 3% $MeOH/CHCl_3$; 100 ml 4% $MeOH/CHCl_3$; 1.3 l 5% MeOH/$CHCl_3$. In elution volution volume 850—1400 ml there is recovered 250 mg of the title compound as a white solid: (45.3% yield): $^{13}C$—NMR ($d_6$-acetone) 138.2, 129.2, 128.4, 94.2, 74.5, 73.1, 67.3, 66.7, 65.2, 57.6, 57.5, 55.9, 50.5, 40.3, 32.5, 31.6, 21.2, 20.9, and 14.2 ppm; exact mass calc'd for $C_{46}H_{78}N_3O_{11}Si_4$ (tetra TMS—$CH_3$) 960.4713, found 960.4722.

Example 49

N,N'-dibenzyloxycarbonyl-3'-(S)-3'-(N-octylaminomethyl)dihydrospectinomycin (Formula $XXI_b$).

Refer to Chart D.

In 5 ml of MeOH are dissolved 500 mg (0.79 mmol) of N,N'-dibenzyloxycarbonyl-3'-(S)-3'-(aminomethyl)dihydrospectinomycin (Example 34). To this solution are added 1.23 ml of n-octanal and a drop of methyl orange/MeOH solution. Then 17 mg of $NaCNBH_3$ in 1 ml of MeOH are added. The pH is lowered to 4 with 1N HCl/MeOH. The reaction is stirred for 1 hr, maintaining the pH at 4. The MeOH is then removed in vacuo and the residue is taken up in $CHCl_3$. The solution is washed with 50 ml of brine and dried over $MgSO_4$. After filtering, the solvent is removed to afford an oil. The product is taken up in $CHCl_3$ and chromatographed on 30 g of silica, slurry packed in $CHCl_3$. The column is eluted as follows: 200 ml $CHCl_3$; 500 ml 1% $MeOH/CHCl_3$; 1 l 2% $MeOH/CHCl_3$; 500 ml 3% $MeOH/CHCl_3$; 1 l 5% $MeOH/CHCl_3$. In elution volume 1100—1400 ml there is recovered 186 mg of the title compound as a white solid (32% yield): $^{13}C$—NMR ($d_6$-acetone) 138.2, 129.2, 128.4, 94.2, 74.5, 74.2, 74.1, 72.9, 67.3, 66.7, 66.5, 65.2, 61.4, 61.1, 60.6, 60.5, 57.6, 57.4, 55.8, 50.8, 40.4, 32.4, 31.5, 30.8, 30.3, 30.1, 29.9, 27.8, 23.2, 21.2, and 14.3 ppm; exact mass calc'd for $C_{51}H_{89}N_3O_{11}Si_4$ (tetra TMS) 1031.5574, found 1031.5585.

Example 50

3'-(S)-3'-(N-ethylaminomethyl)dihydrospectinomycin trihydrochloride

Refer to Chart D.

In 10 ml of MeOH are combined 294 mg (0.44 mmol) of N,N'-dibenzyloxycarbonyl-3'-(s)-3'-(N-ethylaminomethyl)dihydrospectinomycin (Example 47) and 200 mg of Pd black. To this solution are added 170 µl of formic acid. The reaction is stirred for 40 minutes and is filtered. Removal of the solvent in vacuo affords 225 mg of a white solid. The product is dissolved in 2 ml of $H_2O$ and 1.5 ml of 1N HCl is added. The solution is frozen and lyophilized to afford 215 mg of a white solid (97.5% yield): $^{13}C$—NMR ($D_2O$) 96.3, 95.1, 75.5, 74.2, 70.6, 70.5, 70.3, 65.5, 65.0, 62.2, 54.5, 47.6, 41.7, 35.3, 34.6, 23.2, and 13.79 ppm; exact mass calc'd for $C_{35}H_{81}N_3O_7Si_6$ (hexa TMS) 823.4690, found 823,4690.

Example 51

3'-(S)-3'-(N-butylaminomethyl)dihydrospectinomycin trihydrochloride

Refer to Chart D.

In 10 ml of MeOH are dissolved 245 mg (0.39 mmol) of N,N'-dibenzyloxycarbonyl-3'-(S)-3'-(N-butylaminomethyl)dihydrospectinomycin (Example 48). To this solution are added 250 mg of Pd black followed by 152 µl of HCOOH. The reaction is stirred for 45 min and is filtered. Removal of the solvent in vacuo affords 195 mg of a white solid. The material is taken up in 2 ml of $H_2O$ and 1.5 ml of 1N HCl is added. The solution is frozen and lyophilized to afford 166 mg of a white solid (80% yield): $^{13}C$—NMR ($D_2O$) 96.2, 95.3, 75.6, 73.4, 70.8, 69.7, 69.5, 65.1, 63.9, 61.7, 54.9, 52.4, 41.7, 34.8, 34.6, 30.8, 23.4, 22.9, and 16.6 ppm; exact mass calc'd for $C_{37}H_{85}N_3O_7Si_6$ (Hexas-TMS) 851.5003, found 851.4980; exact mass calc'd for $C_{36}H_{82}N_3O_7Si_6$ (Hexa-TMS-$CH_3$) 836.4768, found 836.4757.

Example 52

3'-(S)-3'-(N-octylaminomethyl)dihydrospectinomycin trihydrochloride

Refer to Chart F.

In 5 ml of MeOH is dissolved 180 mg of N,N'-dibenzyloxycarbonyl-3'-(S)-3'-(N-octylaminomethyl)-dihydrospectinomycin (Example 49). To this solution are added 200 mg of Pd black followed by 94 µl of HCOOH. The reaction is stirred for 30 min and is filtered. The solvent is removed in vacuo to afford 150 mg of a white solid. The product is taken up in 2 ml of $H_2O$ and 0.8 ml of 1N HCl is added. The solution is frozen and lyophilized overnight to afford 135 mg of a white solid (96% yield): $^{13}C$—NMR ($D_2O$) 96.2, 95.3, 75.6, 73.4, 70.6, 69.7, 69.5, 65.1, 63.9, 61.7, 54.9, 50.7, 41.8, 34.8, 34.6, 32.0, 29.5, 28.8, 25.8, 23.4, and 17.2 ppm; exact mass calc'd for $C_{41}H_{93}N_3O_7Si_6$ (hexa TMS) 907.5629, found 907.5618.

Example 53

Procedure for the Preparation of 3-(R)-3'-(N-alkylated)dihydrospectinomycins

A. For predominant monoalkylation, N,N'-dibenzyloxycarbonyl-3'-(R)-3'-(aminomethyl)-dihydrospectinomycin (Example 36) is dissolved in MeOH. To the solution are added 10 equivalents of the appropriate aldehyde and a trace of methyl orange indicator. The pH is then adjusted to the indicator's endpoint with methanolic HCl and 0.33 equivalents of $NaCNBH_3$ in MeOH is added. The reaction is stirred at room temperature and the pH is maintained near the endpoint of methyl orange by adding additional methanolic HCl. The reaction is easily followed by TLC using Analtech Silica plates and eluting with 10% $MeOH/CHCl_3$/1% $NH_4OH$ as a solvent. When the reaction is complete, the solvent is removed in vacuo and the residue is partitioned between $H_2O$ and EtOAc. The aqueous phase is made alkaline with the addition of concentrated $NH_4OH$ and the EtOAc is separated and combined with an additional ETOAc extract. The combined extracts are washed with brine and dried over $MgSO_4$. The solvent is then removed in vacuo to afford the crude product. The product is taken up in $CHCl_3$ and chromatographed on silica, slurry packed in $CHCl_3$. The column is eluted with a $MeOH/CHCl_3$ gradient

(1%—5%), and 50 ml fractions are taken. The fractions are analyzed by TLC and pure fractions are pooled and concentrated in vacuo to afford the product.

B. For predominant dialkylation, the above procedure is followed using 0.66 equivalents of $NaCNBH_3$.

According to the procedure above, there are prepared:

(a) N,N′-dibenzyloxycarbonyl-3′-(R)-3′-(N-ethylaminomethyl)-dihydrospectinomycin: Alkylation of 491 mg (0.78 mmol) of N,N′-dibenzyloxycarbonyl-3′-(R)-3′-(aminomethyl)dihydrospectinomycin affords 123 mg (24% yield) of the product, after chromatography: $^{13}C$—NMR ($d_6$-acetone) 138.1, 129.1, 128.3, 128.1, 97.0, 93.8, 74.5, 71.6, 67.1, 66.4, 65.2, 57.6, 54.0, 44.4, 43.8, 31.6, 21.5 and 14.9 ppm; exact mass calc'd for $C_{45}H_{77}N_3O_{11}Si_4$ (tetra TMS) 947.4653, found 947.4601.

(b) N,N′-dibenzyloxycarbonyl-3′-(R)-3′-(N,N-diisobutylaminomethyl)dihydrospectinomycin: Dialkylation of 631 mg (1 mmol) of amine affords 171 mg of the product as a white solid (23% yield): $^{13}C$—NMR ($d_6$-acetone) 138.1, 129.1, 128.3, 128.1, 97.0, 93.5, 74.6, 73.1, 67.3, 67.2, 66.4, 65.2, 61.1, 57.5, 44.1, 31.4, 26.8, 21.7, and 21.4 ppm; exact mass calc'd for $C_{48}H_{81}N_3O_1Si_4$ (tri-TMS) 959.5179, found 959.5134.

## Example 54

Deprotection of N,N′-dibenzyloxycarbonyl-3′-(R)-3′-N-mono and N,N-dialkylaminomethyl dihydrospectinomycins

The protected substrate is dissolved in MeOH and an equivalent weight of Pd black is added. To this mixture are added 10 equivalents of formic acid. The reaction is stirred for 45 min, filtered and concentrated to dryness. The residue is taken up in $H_2O$ and 3.5 equivalents of 1N HCl are added. The solution is frozen and lyophilized to afford the final product.

Accordingly, the products of Example 53 yield:

(a) 3′-(R)-3′-(N-ethylaminomethyl)dihydrospectinomycin trihydrochloride: Deprotection of 123 mg (0.19 mmol) affords 83 mg (89% yield) of the product: $^{13}C$—NMR ($D_2O$) 94.0, 93.2, 73.2, 70.4, 68.2, 66.8, 66.2, 62.4, 60.5, 59.3, 50.5, 45.1, 40.7, 31.9, 31.5, 21.0 and 11.1 ppm; exact mass calc'd for $C_{35}H_{81}N_3O_{11}Si_6$ (hexa-TMS) 823.4690, found 823.4665.

(b) 3′-(R)-3′-(N,N′-diisobutylaminomethyl)dihydrospectinomycin trihydrochloride: Deprotection of 153 mg (0.20 mmol) affords 96 mg of the product (82% yield): $^{13}C$—NMR ($D_2O$) 94.3, 93.9, 73.3, 70.6, 68.2, 66.7, 66.6, 65.7, 62.6, 60.7, 59.6, 43.3, 31.9, 31.8, 31.6, 25.1, 24.8, 21.1, 21.0, 20.6, 20.4, 19.8 and 19.6 ppm; exact mass calc'd for $C_{38}H_{85}N_3O_7Si_5$ (penta-TMS) 835.5234, found 835.5241.

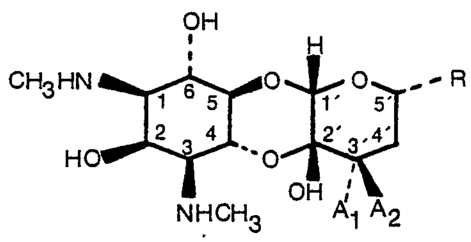

I

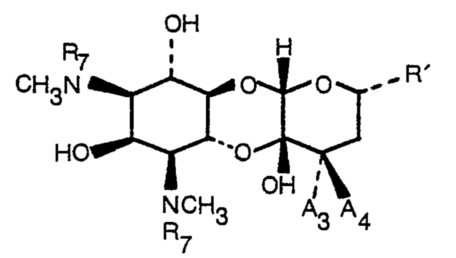

$II_b$

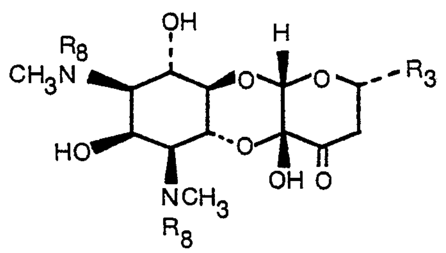

$III_{(b)}$

CHART A

3'C =O  $III_b$

3'C =N-NHR$_{60}$  $IV_b$

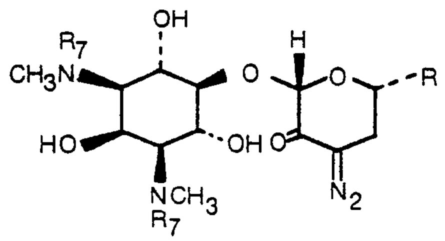

$V_b$

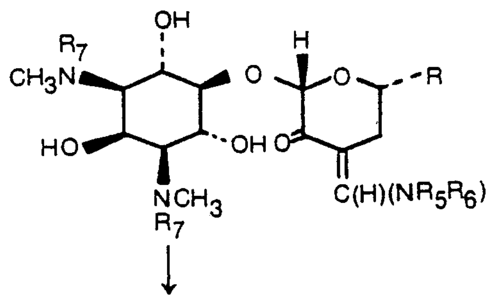

$VI_b$

3'C =CH$_2$  $VII_b$

+

3' (H)(CH$_2$NR$_5$R$_6$)  $VIII_b$

CHART B

3'C =CH$_2$  $VII_b$

3'C β-O-CH$_2$-α  $IX_b$

3'C β-OH:α-CH$_2$N$_3$  $XI_b$

3'C β-OH:α-CH$_2$NR$_1$R$_2$  $XIII_b$ → XIII

3'C β-OH:α-CH$_2$CN  $XV_b$ → XV

CHART C

| | | |
|---|---|---|
| 3'C | $=CH_2$ | $VII_b$ |
| ↓ | | |
| 3'C | $\beta-CH_2-O-\alpha$ | $X_b$ |
| ↓ | | |
| 3'C | $\alpha-OH:\beta-CH_2N_3$ | $XII_b$ |
| 3'C | $\alpha-OH:\beta-CH_2NR_1R_2$ | $XIV_b$ → XIV |
| 3'C | $\alpha-OH:\beta-CH_2CN$ | $XVI_b$ → XVI |

CHART D

| | | | |
|---|---|---|---|
| | 3'C | =O | III(b) |
| | ↓ | | |
| XVIII(b) → | 3'C | $\beta-OH:\alpha-CN$ | XVII(b) |
| | ↓ | | |
| $XI_b$ → | 3'C | $\beta-OH:\alpha-CH_2NH_2$ | XIX(b) |
| | ↓ | | |
| | 3'C | $\beta-OH:\alpha-CH_2NR_1R'_2$ | $XXI_b$ → XXI |
| | 3'C | $\beta-OH:\alpha-CH_2NHCOR_4$ | XXIII(b) |

CHART E

3'C =O — III(b)

↓

3'C α-OH:β-CN — XVIII(b)

↓

$XII_b$ → 3'C α-OH:β-$CH_2NH_2$ — XX(b)

↓

3'C α-OH:β-$CH_2NR_1R'_2$ — $XXII_b$ → XXII

3'C α-OH:β-$CH_2NHCOR_4$ — XXIV(b)

**Claims**

1. A compound of formula I

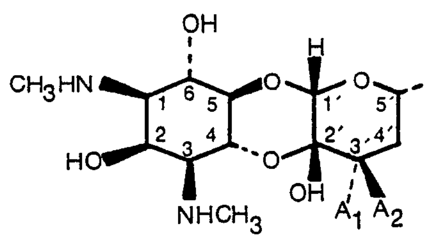

I

wherein

R is H, $C_{1-8}$ alkyl, $C_{1-4}$ hydroxyalkyl, ($C_{1-6}$alkoxy)$C_{1-4}$ alkyl having up to 7 C atoms in total, $C_{1-4}$ aminoalkyl, ($C_{1-6}$alkylamino)$C_{1-4}$ alkyl having up to 7 C atoms in total, [di($C_{1-6}$ alkyl)amino]$C_{1-4}$ alkyl in which the $C_{1-4}$ alkyl group and either $C_{1-6}$ alkyl group together have up to 7 C atoms, $C_{1-4}$ haloalkyl, $C_{1-4}$ dihaloalkyl or $C_{1-4}$ trihaloalkyl; and

one of $A_1$ and $A_2$ is OH and the other is CN, $CH_2CN$ or $CH_2NR_1R_2$ in which either $NR_1R_2$ is a heterocyclic radical having 3 to 10 ring atoms or $R_1$ and $R_2$ are independently selected from H, $C_{1-12}$ alkyl, $C_{6-12}$ aryl, optionally mono- or di- ring-substituted $C_{7-12}$ aralkyl, $C_{3-10}$ cycloalkyl, ($C_{3-10}$ cycloalkyl)$C_{1-4}$ alkyl and acyl selected from ($C_{1-12}$ alkyl)carbonyl, (optionally mono- or di-ring-substituted $C_{7-12}$ aralkyl)carbonyl, ($C_{3-10}$ cycloalkyl)carbonyl, [($C_{3-10}$ cycloalkyl)$C_{1-4}$ alkyl]carbonyl and optionally ring-substituted pyridylcarbonyl, piperazylcarbonyl, pyrrolylcarbonyl or morpholinocarbonyl, and any ring substituents are independently selected from F, Cl, I, $NH_2$, $C_{1-6}$ alkylamino, di($C_{1-6}$ alkyl)amino, CN, OH, COOH, ($C_{1-4}$ alkyl)carbonyl and $C_{1-4}$ alkoxy;

or a pharmacologically-acceptable acid addition salt thereof.

2. A compound as claimed in claim 1, wherein $A_1$ or $A_2$ is $CH_2NR_1R_2$ and either $NR_1R_2$ is a heterocyclic radical having 3 to 10 ring atoms or $R_1$ and $R_2$ are independently selected from H, $C_{1-6}$ alkyl, $C_{3-10}$ cycloalkyl or ($C_{3-10}$ cycloalkyl)$C_{1-4}$ alkyl.

3. A compound as claimed in claim 1, wherein $A_1$ or $A_2$ is $CH_2NR_1R_2$ and $R_1$ and $R_2$ are independently selected from $C_{6-12}$ aryl, optionally ring-substituted $C_{7-12}$ aralkyl and acyl.

4. A compound selected from

3'-(R)-3'-aminomethyldihydrospectinomycin trihydrochloride;

3'-(S)-3'-aminomethyldihydrospectinomycin trihydrochloride;

3'-(R)-3'-(N-ethylaminomethyl)dihydrospectinomycin trihydrochloride;

3'-(S)-3'-(N-ethylaminomethyl)dihydrospectinomycin trihydrochloride;
3'-(R)-3'-(N-propylaminomethyl)dihydrospectinomycin trihydrochloride;
3'-(S)-3'-(N-propylaminomethyl)dihydrospectinomycin trihydrochloride;
3'-(R)-3'-(N-butylaminomethyl)dihydrospectinomycin trihydrochloride;
3'-(S)-3'-(N-butylaminomethyl)dihydrospectinomycin trihydrochloride;
3'-(R)-3'-(N-isobutylaminomethyl)dihydrospectinomycin trihydrochloride;
3'-(R)-3'-(N-pentylaminomethyl)dihydrospectinomycin trihydrochloride;
3'-(R)-3'-[N-(2-methylbutyl)aminomethyl]dihydrospectinomycin trihydrochloride;
3'-(R)-3'-(N-hexylaminomethyl)dihydrospectinomycin trihydrochloride;
3'-(R)-3'-(N-octylaminomethyl)dihydrospectinomycin trihydrochloride;
3'-(S)-3'-(N-octylaminomethyl)dihydrospectinomycin trihydrochloride;
3'-(R)-3'-(N,N-diisobutylaminomethyl)dihydrospectinomycin trihydrochloride; and
3'-(R)-3'-(N,N-dihexylaminomethyl)dihydrospectinomycin trihydrochloride.

5. A compound selected from
3'-(R)-3'-(N-cyclopentylaminomethyl)dihydrospectinomycin trihydrochloride;
3'-(R)-3'-(N-cyclohexylaminomethyl)dihydrospectinomycin trihydrochloride;
3'-(R)-3'-(N-cycloheptylaminomethyl)dihydrospectinomycin trihydrochloride;
3'-(R)-3'-[N-(cyclohexylmethyl)aminomethyl]dihydrospectinomycin trihydrochloride;
3'-(R)-3'-[N-(cyclooctylmethyl)aminomethyl]dihydrospectinomycin trihydrochloride; and
3'-(R)-3'-[N,N-di(cyclohexylmethyl)aminomethyl]dihydrospectinomycin trihydrochloride.

6. A compound selected from
3'-(R)-3'-(N-acetylaminomethyl)dihydrospectinomycin dihydrochloride;
3'-(R)-3'-(N-glycylaminomethyl)dihydrospectinomycin trihydrochloride;
3'-(R)-3'-[N-(pyrrole-2-carbonyl)aminomethyl]dihydrospectinomycin dihydrochloride; and
3'-(R)-3'-[N-(succinyl)aminomethyl]dihydrospectinomycin trihydrochloride.

7. 3'-(Cyanomethyl)dihydrospectinomycin dihydrochloride.

8. A compound of formula $II_b$

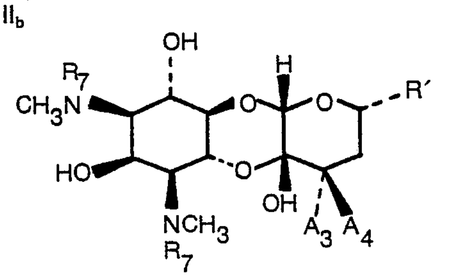

$II_b$

wherein $R_7$ is a blocking group;

R' is H, $C_{1-8}$ alkyl, $C_{1-4}$ hydroxyalkyl, $(C_{1-6}alkoxy)C_{1-4}$ alkyl having up to 7 C atoms in total, N-blocked $C_{1-4}$ aminoalkyl, N-blocked $(C_{1-6}$ alkylamino$(C_{1-4}$ alkyl, $[di(C_{1-4}$ alkyl)amino$]C_{1-4}$ alkyl in which the $C_{1-4}$ alkyl group and either $C_{1-6}$ alkyl group together have up to 7 C atoms, $C_{1-4}$ haloalkyl, $C_{1-4}$ dihaloalkyl or $C_{1-4}$ trihaloalkyl; and

either $A_3$ and $A_4$ are as defined for $A_1$ and $A_2$ in claim 1, or one of $A_3$ and $A_4$ is OH and the other is $CH_2N_3$.

9. A compound selected from
N,N'-dibenzyloxycarbonyl-3'-(R)-3'-aminomethyldihydrospectinomycin;
N,N'-dibenzyloxycarbonyl-3'-(S)-3'-aminomethyldihydrospectinomycin;
N,N'-dibenzyloxycarbonyl-3'-(R)-3'-(N''-ethylaminomethyl)dihydrospectinomycin;
N,N'-dibenzyloxycarbonyl-3'-(S)-3'-(N''-ethylaminomethyl)dihydrospectinomycin;
N,N'-dibenzyloxycarbonyl-3'-(R)-3'-(N''-propylaminomethyl)dihydrospectinomycin;
N,N'-dibenzyloxycarbonyl-3'-(R)-3'-(N''-butylaminomethyl)dihydrospectinomycin;
N,N'-dibenzyloxycarbonyl-3'-(S)-3'-(N''-butylaminomethyl)dihydrospectinomycin;
N,N'-dibenzyloxycarbonyl-3'-(R)-3'-(N''-isobutylaminomethyl)dihydrospectinomycin;
N,N'-dibenzyloxycarbonyl-3'-(R)-3'-(N''-pentylaminomethyl)dihydrospectinomycin;
N,N'-dibenzyloxycarbonyl-3'-(R)-3'-[N''-(2-methylbutyl)aminomethyl]dihydrospectinomycin;
N,N'-dibenzyloxycarbonyl-3'-(R)-3'-(N''-hexylaminomethyl)dihydrospectinomycin;
N,N'-dibenzyloxycarbonyl-3'-(R)-3'-(N''-octylaminomethyl)dihydrospectinomycin;
N,N'-dibenzyloxycarbonyl-3'-(S)-3'-(N''-octylaminomethyl)dihydrospectinomycin;
N,N'-dibenzyloxycarbonyl-3'-(R)-3'-(N'',N''-diisobutylaminomethyl)dihydrospectinomycin; and
N,N'-dibenzyloxycarbonyl-3'-(N'',N''-dihexylaminomethyl)dihydrospectinomycin.

10. A compound selected from
N,N'-dibenzyloxycarbonyl-3'-(R)-3'-(N''-cyclopentylaminomethyl)dihydrospectinomycin;
N,N'-dibenzyloxycarbonyl-3'-(R)-3'-(N''-cyclohexylaminomethyl)dihydrospectinomycin;
N,N'-dibenzyloxycarbonyl-3'-(R)-3'-(N''-cycloheptylaminomethyl)dihydrospectinomycin;
N,N'-dibenzyloxycarbonyl-3'-(R)-3'-[N''-(cyclohexylmethyl)aminomethyl]dihydrospectinomycin;
N,N'-dibenzyloxycarbonyl-3'-(R)-3'-[N''-(cyclooctylmethyl)aminomethyl]dihydrospectinomycin; and
N,N'-dibenzyloxycarbonyl-3'-(R)-3'-[N'',N''-di(cyclohexylmethyl)aminomethyl]dihydrospectinomycin.

11. A compound selected from
N,N'-dibenzyloxycarbonyl-3'-(R)-3'-[N''-(benzyloxycarbonylmethyl)aminomethyl]dihydrospectinomycin;
N,N'-dibenzyloxycarbonyl-3'-(R)-3'-(N''-acetylaminomethyl)dihydrospectinomycin; and
N,N'-dibenzyloxycarbonyl-3'-(R)-3'-(N''-succinylaminomethyl)dihydrospectinomycin.

12. A compound selected from
N,N'-dibenzyloxycarbonyl-3'-(azidomethyl)dihydrospectinomycin;
N,N'-dibenzyloxycarbonyl-3'-(cyanomethyl)dihydrospectinomycin; and
N,N'-dibenzyloxycarbonyl-3'-(R)-spectinomycin cyanohydrin.

13. A process for preparing a mixture of epimers of a compound as claimed in claim 1 when $A_1$ or $A_2$ is CN or a mixture of epimers of a compound as claimed in claim 8 when $A_3$ or $A_4$ is CN, which comprises subjecting to hydrocyanation a compound of the formula $III_{(b)}$

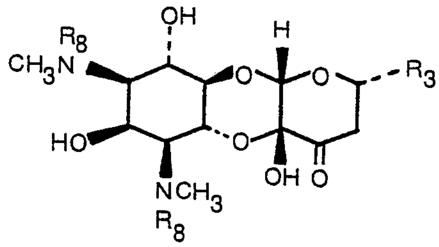

$III_{(b)}$

wherein $R_8$ is H and $R_3$ is R as defined in claim 1 or $R_8$ is a blocking group and $R_3$ is R' as defined in claim 8, in the presence of a base or a basic ion-exchange resin, in an inert organic solvent; and either allowing the reaction product to remain in solution for a time sufficient that, predominantly in the mixture, $A_1$ or $A_3$ is CN; or terminating the reaction when, predominantly in the mixture, $A_2$ or $A_4$ is CN, either by the addition of acid to neutralise the base or basic ion-exchange resin, or, if no other base is present, removing the resin.

**Patentansprüche**

1. Eine Verbindung der Formel I

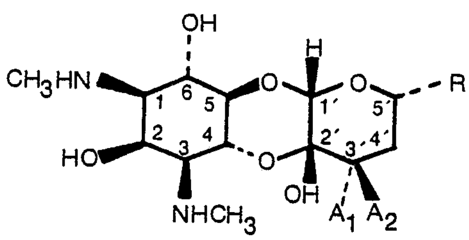

I

worin
R H, $C_{1-8}$ Alkyl, $C_{1-4}$ Hydroxyalkyl, ($C_{1-6}$ Alkoxy)$C_{1-4}$ Alkyl mit bis 7 C Atomen insgesamt, $C_{1-4}$ Aminoalkyl, ($C_{1-6}$ Alkylamino)$C_{1-4}$ Alkyl mit bis 7 C Atomen insgesamt, [di($C_{1-6}$ Alkyl)Amino]$C_{1-4}$ Alkyl, worin die $C_{1-4}$ Alkylgruppe und eine der beiden $C_{1-6}$ Alkylgruppen gemeinsam bis 7 C Atome aufweisen, $C_{1-4}$ Haloalkyl, $C_{1-4}$ Dihaloalkyl oder $C_{1-4}$ Trihaloalkyl ist; und
von $A_1$ und $A_2$ eines OH und das andere CN, $CH_2CN$ oder $CH_2NR_1R_2$ ist, wobei entweder $NR_1R_2$ ein heterocyclisches Radikal mit 3 bis 10 Ringatomen ist oder $R_1$ und $R_2$ unabhängig aus der Gruppe H, $C_{1-12}$ Alkyl, $C_{6-12}$ Aryl, wahlweise mono- oder di-ringsubstituiertes $C_{7-12}$ Aralkyl, $C_{3-10}$ Cycloalkyl, ($C_{3-10}$ Cycloalkyl)$C_{1-4}$ Alkyl und Acyl ausgewählt aus der Gruppe ($C_{1-12}$ Alkyl)Carbonyl, (wahlweise mono- oder di-ringsubstituiertes $C_{7-12}$ Aralkyl)Carbonyl, ($C_{3-10}$ Cycloalkyl) Carbonyl, [($C_{3-10}$ Cycloalkyl)$C_{1-4}$ Alkyl]Carbonyl und wahlweise ring-substituiertes Pyridylcarbonyl, Piperazylcarbonyl, Pyrrolylcarbonyl oder Morpholinocarbonyl unabhängig ausgewählt sind, und etwaige Ring-substituenten unabhängig aus der Gruppe F, Cl, I, $NH_2$, $C_{1-6}$ Alkylamino, di($C_{1-6}$ Alkyl)Amino, CN, OH, COOH, ($C_{1-4}$ Alkyl)Carbonyl und $C_{1-4}$ Alkoxy ausgewählt sind;
oder ein pharmakologisch annehmbares saures Zusatzsalz davon.

2. Eine Verbindung nach Anspruch 1, worin $A_1$ oder $A_2$ $CH_2NR_1R_2$ ist und entweder $NR_1R_2$ ein heterocyclisches Radikal mit 3 bis 10 Ringatomen ist oder $R_1$ und $R_2$ unabhängig aus der Gruppe H, $C_{1-6}$ Alkyl, $C_{3-10}$ Cycloalkyl oder ($C_{3-10}$ Cycloalkyl)$C_{1-4}$ Alkyl ausgewählt sind.

3. Eine Verbindung im Einklang mit Anspruch 1, worin $A_1$ oder $A_2$ $CH_2NR_1R_2$ ist und $R_1$ und $R_2$ unabhängig aus der Gruppe $C_{6-12}$ Aryl, wahlweise ringsubstituiertes $C_{7-12}$ Aralkyl und Acyl ausgewählt sind.

4. Eine Verbindung ausgewählt aus der Gruppe
3'-(R)-3'-Aminomethyldihydrospectinomycin-Trihydrochlorid;
3'-(S)-3'-Aminomethyldihydrospectinomycin-Trihydrochlorid;

3'-(R)-3'-(N-Äthylaminomethyl)Dihydrospectinomycin-Trihydrochlorid;
3'-(S)-3'-(N-Äthylaminomethyl)Dihydrospectinomycin-Trihydrochlorid;
3'-(R)-3'-(N-Propylaminomethyl)Dihydrospectinomycin-Trihydrochlorid;
3'-(S)-3'-(N-Propylaminomethyl)Dihydrospectinomycin-Trihydrochlorid;
3'-(R)-3'-(N-Butylaminomethyl)Dihydrospectinomycin-Trihydrochlorid;
3'-(S)-3'-(N-Butylaminomethyl)Dihydrospectinomycin-Trihydrochlorid;
3'-(R)-3'-(N-Isobutylaminomethyl)Dihydrospectinomycin-Trihydrochlorid;
3'-(R)-3'-(N-Pentylaminomethyl)Dihydrospectinomycin-Trihydrochlorid;
3'-(R)-3'-[N-(2-Methylbutyl)Aminomethyl]Dihydrospectinomycin-Trihydrochlorid;
3'-(R)-3'-(N-Hexylaminomethyl)Dihydrospectinomycin-Trihydrochlorid;
3'-(R)-3'-(N-Octylaminomethyl)Dihydrospectinomycin-Trihydrochlorid;
3'-(S)-3'-(N-Octylaminomethyl)Dihydrospectinomycin-Trihydrochlorid;
3'-(R)-3'-(N,N-Disobutylaminomethyl)Dihydrospectinomycin-Trihydrochlorid; und
3'-(R)-3'-(N,N-Dihexylaminomethyl)Dihydrospectinomycin-Trihydrochlorid.

5. Eine Verbindung ausgewählt aus der Gruppe
3'-(R)-3'-(N-Cyclopentylaminomethyl)Dihydrospectinomycin-Trihydrochlorid;
3'-(R)-3'-(N-Cyclohexylaminomethyl)Dihydrospectinomycin-Trihydrochlorid;
3'-(R)-3'-(N-Cycloheptylaminomethyl)Dihydrospectinomycin-Trihydrochlorid;
3'-(R)-3'-[N-(Cyclohexylmethyl)Aminomethyl]Dihydrospectinomycin-Trihydrochlorid;
3'-(R)-3'-[N-(Cyclooctylmethyl)Aminomethyl]Dihydrospectinomycin-Trihydrochlorid; und
3'-(R)-3'-[N,N-di(Cyclohexylmethyl)Aminomethyl]Dihydrospectinomycin-Trihydrochlorid.

6. Eine Verbindung ausgewählt aus der Gruppe
3'-(R)-3'-(N-Acetylaminomethyl)Dihydrospectinomycin-Dihydrochlorid;
3'-(R)-3'-(N-Glycylaminomethyl)Dihydrospectinomycin-Trihydrochlorid;
3'-(R)-3'-[N-(Pyrrol-2-Carbonyl)Aminomethyl]Dihydrospectinomycin-Dihydrochlorid; und
3'-(R)-3'-[N-(Succinyl)Aminomethyl]Dihydrospectinomycin-Trihydrochlorid.

7. 3'-(Cyanomethyl)Dihydrospectinomycin-Dihydrochlorid.

8. Eine Verbindung der Formel $II_b$

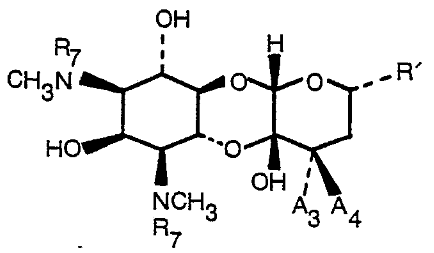

$II_b$

worin $R_7$ eine blockierende Gruppe ist;

R' H, $C_{1-8}$ Alkyl, $C_{1-4}$ Hydroxyalkyl, $(C_{1-6}$ Alkoxy$)C_{1-4}$ Alkyl mit bis 7 C Atomen insgesamt, N-blockiertes $C_{1-4}$ Aminoalkyl, N-blockiertes $(C_{1-6}$ Alkylamino$)C_{1-4}$ Alkyl, [di$(C_{1-4}$ Alkyl)Amino]$C_{1-4}$ Alkyl, wobei die $C_{1-4}$ Alkylgruppe und eine der beiden $C_{1-6}$ Alkylgruppen gemeinsam bis 7 C Atome aufweisen, $C_{1-4}$ Haloalkyl, $C_{1-4}$ Dihaloalkyl oder $C_{1-4}$ Trihaloalkyl ist; und

entweder $A_3$ und $A_4$ wie für $A_1$ und $A_2$ in Anspruch 1 definiert sind oder von $A_3$ und $A_4$ eines OH und das andere $CH_2N_3$ ist.

9. Eine Verbindung ausgewählt aus der Gruppe
N,N'-Dibenzyloxycarbonyl-3'-(R)-3'-Aminomethyldihydrospectinomycin;
N,N'-Dibenzyloxycarbonyl-3'-(S)-3'-Aminomethyldihydrospectinomycin;
N,N'-Dibenzyloxycarbonyl-3'-(R)-3'-(N''-Äthylaminomethyl)Dihydrospectinomycin;
N,N'-Dibenzyloxycarbonyl-3'-(R)-3'-(N''-Äthylaminomethyl)Dihydrospectinomycin;
N,N'-Dibenzyloxycarbonyl-3'-(R)-3'-(N''-Propylaminomethyl)Dihydrospectinomycin;
N,N'-Dibenzyloxycarbonyl-3'-(R)-3'-(N''-Butylaminomethyl)Dihydrospectinomycin;
N,N'-Dibenzyloxycarbonyl-3'-(S)-3'-(N''-Butylaminomethyl)Dihydrospectinomycin;
N,N'-Dibenzyloxycarbonyl-3'-(R)-3'-(N''-Isobutylaminomethyl)Dihydrospectinomycin;
N,N'-Dibenzyloxycarbonyl-3'-(R)-3'-(N''-Pentylaminomethyl)Dihydrospectinomycin;
N,N'-Dibenzyloxycarbonyl-3'-(R)-3'-[N''-(2-Methylbutyl)Aminomethyl]Dihydrospectinomycin;
N,N'-Dibenzyloxycarbonyl-3'-(R)-3'-(N''-Hexylaminomethyl)Dihydrospectinomycin;
N,N'-Dibenzyloxycarbonyl-3'-(R)-3'-(N''-Octylaminomethyl)Dihydrospectinomycin;
N,N'-Dibenzyloxycarbonyl-3'-(S)-3'-(N''-Octylaminomethyl)Dihydrospectinomycin;
N,N'-Dibenzyloxycarbonyl-3'-(R)-3'-(N'',N''-Diisobutylaminomethyl)Dihydrospectinomycin; und
N,N'-Dibenzyloxycarbonyl-3'-(N'',N''-Dihexylaminomethyl)Dihydrospectinomycin.

10. Eine Verbindung ausgewählt aus der Gruppe
N,N'-Dibenzyloxycarbonyl-3'-(R)-3'-(N''-Cyclopentylaminomethyl)Dihydrospectinomycin;
N,N'-Dibenzyloxycarbonyl-3'-(R)-3'-(N''-Cyclohexylaminomethyl)Dihydrospectinomycin;
N,N'-Dibenzyloxycarbonyl-3'-(R)-3'-(N''-Cycloheptylaminomethyl)Dihydrospectinomycin;

N,N'-Dibenzyloxycarbonyl-3'-(R)-3'-[N''-(Cyclohexylmethyl)Aminomethyl]Dihydrospectinomycin;
N,N'-Dibenzyloxycarbonyl-3'-(R)-3'-[N''-(Cyclooctylmethyl)Aminomethyl]Dihydrospectinomycin; und
N,N'-Dibenzyloxycarbonyl-3'-(R)-3'-[N'',N''-di(Cyclohexylmethyl)Aminomethyl]-Dihydrospectinomycin.

11. Eine Verbindung ausgewählt aus der Gruppe
N,N'-Dibenzyloxycarbonyl-3'-(R)-3'-[N''-(Benzyloxycarbonylmethyl)Aminomethyl]-Dihydrospectinomyzin;
N,N'-Dibenzyloxycarbonyl-3'-(R)-3'-(N''-Acetylaminomethyl)Dihydrospectinomycin; und
N,N'-Dibenzyloxycarbonyl-3'-(R)-3'-(N''-Succinylaminomethyl)Dihydrospectinomycin.

12. Eine Verbindung ausgewählt aus der Gruppe
N,N'-Dibenzyloxycarbonyl-3'-(Azidomethyl)Dihydrospectinomycin;
N,N'-Dibenzyloxycarbonyl-3'-(Cyanomethyl)Dihydrospectinomycin; und
N,N'-Dibenzyloxycarbonyl-3'-(R)-Spectinomycincyanohydrin.

13. Ein Verfahren zur Herstellung eines Gemisches von Epimeren einer Verbindung nach Anspruch 1, bei der $A_1$ oder $A_2$ CN oder ein Gemisch von Epimeren einer Verbindung nach Anspruch 8 ist, wenn $A_3$ oder $A_4$ CN ist, das sich auf die Hydrocyanierung einer Verbindung der Formel $III_{(b)}$ erstreckt

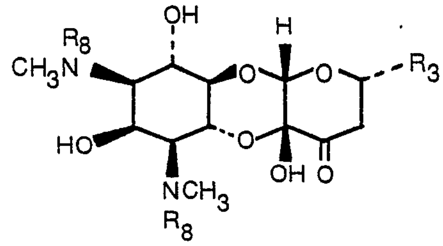

$III_{(b)}$

worin $R_8$ H und $R_3$ R wie in Anspruch 1 definiert ist, oder $R_8$ eine blockierende Gruppe und $R_3$ R' wie in Anspruch 8 definiert ist, in Gegenwart einer Base oder eines basischen Ionenaustauschharzes in einem inerten organischen Lösungsmittel; wobei entweder das Reaktionsprodukt solange in Lösung belassen wird, bis $A_1$ oder $A_3$, vorwiegend in dem Gemisch, CN ist; oder die Reaktion beendet wird, wenn $A_2$ oder $A_4$, vorwiegend in dem Gemisch, CN ist, und zwar entweder durch den Zusatz von Säure zwecks Neutralisierung der Base oder des basischen Ionenaustauschharzes oder, falls keine andere Base vorhanden ist, durch Entfernen des Harzes.

**Revendications**

1. Composé de formule I

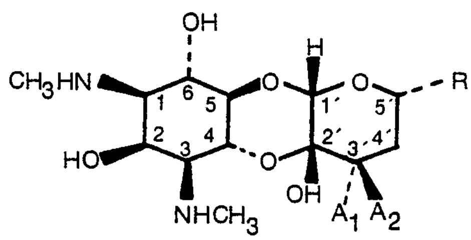

I

dans laquelle

R représente H, un groupe alkyle en $C_1$ à $C_8$, hydroxyalkyle en $C_1$ à $C_4$, (alkoxy en $C_1$ à $C_6$)-alkyle en $C_1$ à $C_4$ ayant au total jusqu'à 7 atomes de carbone, aminoalkyle en $C_1$ à $C_4$, (alkylamino en $C_1$ à $C_6$)-alkyle en $C_1$ à $C_4$ ayant au total jusqu'à 7 atomes de carbone, [di(alkyle en $C_1$ à $C_6$)amino]-alkyle en $C_1$ à $C_4$ dans lequel le groupe alkyle en $C_1$ à $C_4$ et chaque groupe alkyle en $C_1$ à $C_6$ possèdent conjointement jusqu'à 7 atomes de carbone, halogénalkyle en $C_1$ à $C_4$, dihalogénalkyle en $C_1$ à $C_4$ ou trihalogénalkyle en $C_1$ à $C_4$; et

l'un des substituants $A_1$ et $A_2$ représente un groupe OH et l'autre représente un groupe CN, $CH_2CN$ ou $CH_2NR_1R_2$ dans lequel $NR_1R_2$ est un radical hétérocyclique ayant 3 à 10 atomes de carbone dans le noyau ou bien $R_1$ et $R_2$ sont choisis indépendamment entre H, un groupe alkyle en $C_1$ à $C_{12}$, aryle en $C_6$ à $C_{12}$, aralkyle en $C_7$ à $C_{12}$, facultativement mono- ou di-substitué sur le noyau, cycloalkyle en $C_3$ à $C_{10}$, (cycloalkyle en $C_3$ à $C_{10}$)-alkyle en $C_1$ à $C_4$ et acyle choisi entre un groupe (alkyle en $C_1$ à $C_{12}$) carbonyle, (aralkyle en $C_7$ à $C_{12}$ facultativement mono- ou di-substitué sur le noyau)- carbonyle, (cycloalkyle en $C_3$ à $C_{10}$)carbonyle, [(cycloalkyle en $C_3$ à $C_{10}$)-alkyle en $C_1$ à $C_4$]carbonyle et pyridylcarbonyle facultativement substitué sur le noyau, pipérazylcarbonyle, pyrrolylcarbonyle ou morpholinocarbonyle, et tous les substituants sur le noyau sont choisis indépendamment entre F, Cl, I, $NH_2$, un groupe alkylamino en $C_1$ à $C_6$, di(alkyle en $C_1$ à $C_6$)amino, CN, OH, COOH, (alkyle en $C_1$ à $C_4$)carbonyle et alkoxy en $C_1$ à $C_4$;

ou un de ses sels d'addition d'acides pharmacologiquement acceptables.

2. Composé suivant la revendication 1, dans lequel $A_1$ ou $A_2$ représente un groupe $CH_2NR_1R_2$ et $NR_1R_2$ est un radical hétérocyclique ayant 3 à 10 atomes dans le noyau ou bien $R_1$ et $R_2$ sont choisis indépendamment entre H, un groupe alkyle en $C_1$ à $C_6$, cycloalkyle en $C_3$ à $C_{10}$ ou (cycloalkyle en $C_3$ à $C_{10}$)-alkyle en $C_1$ à $C_4$.

3. Composé suivant la revendication 1, dans lequel $A_1$ ou $A_2$ représente un groupe $CH_2NR_1R_2$ et $R_1$ et $R_2$ sont choisis indépendamment entre un groupe aryle en $C_6$ à $C_{12}$, aralkyle en $C_7$ à $C_{12}$ facultativement substitué sur le noyau et acyle.

4. Composé choisi entre
le trichlorhydrate de 3'-(R)-3'-aminométhyldihydrospectinomycine;
le trichlorhydrate de 3'-(S)-3'-aminométhyldihydrospectinomycine;
le trichlorhydrate de 3'-(R)-3'-(N-éthylaminométhyl)dihydrospectinomycine;
le trichlorhydrate de 3'-(S)-3'-(N-éthylaminométhyl)dihydrospectinomycine;
le trichlorhydrate de 3'-(R)-3'-(N-propylaminométhyl)dihydrospectinomycine;
le trichlorhydrate de 3'-(S)-3'-(N-propylaminométhyl)dihydrospectinomycine;
le trichlorhydrate de 3'-(R)-3'-(N-butylaminométhyl)dihydrospectinomycine;
le trichlorhydrate de 3'-(S)-3'-(N-butylaminométhyl)dihydrospectinomycine;
le trichlorhydrate de 3'-(R)-3'-(N-isobutylaminométhyl)dihydrospectinomycine;
le trichlorhydrate de 3'-(R)-3'-(N-pentylaminométhyl)dihydrospectinomycine;
le trichlorhydrate de 3'-(R)-3'-[N-(2-méthylbutyl)-aminométhyl]dihydrospectinomycine;
le trichlorhydrate de 3'-(R)-3'-(N-hexylaminométhyl)dihydrospectinomycine;
le trichlorhydrate de 3'-(R)-3'-(N-octylaminométhyl)dihydrospectinomycine;
le trichlorhydrate de 3'-(S)-3'-(N-octylaminométhyl)dihydrospectinomycine;
le trichlorhydrate de 3'-(R)-3'-(N,N-diisobutylaminométhyl)dihydrospectinomycine; et
le trichlorhydrate de 3'-(R)-3'-(N,N-dihexylaminométhyl)dihydrospectinomycine.

5. Composé choisi entre
le trichlorhydrate de 3'-(R)-3'-(N-cyclopentylaminométhyl)dihydrospectinomycine;
le trichlorhydrate de 3'-(R)-3'-(N-cyclohexylaminométhyl)dihydrospectinomycine;
le trichlorhydrate de 3'-(R)-3'-(N-cycloheptylaminométhyl)dihydrospectinomycine;
le trichlorhydrate de 3'-(R)-3'-[N-(cyclohexylméthyl)aminométhyl]dihydrospectinomycine;
le trichlorhydrate de 3'-(R)-3'-[N-(cyclooctylméthyl)aminométhyl]dihydrospectinomycine; et
le trichlorhydrate de 3'-(R)-3'-[N,N-di(cyclohexylméthyl)aminométhyl]dihydrospectinomycine.

6. Composé choisi entre
le dichlorhydrate de 3'-(R)-3'-(N-acétylaminométhyl)dihydrospectinomycine;
le trichlorhydrate de 3'-(R)-3'-(N-glycylaminométhyl)dihydrospectinomycine;
le dichlorhydrate de 3'-(R)-3'-[N-(pyrrole-2-carbonyl)aminométhyl]dihydrospectinomycine; et
le trichlorhydrate de 3'-(R)-3'-[N-(succinyl)aminométhyl]dihydrospectinomycine.

7. Dichlorhydrate de 3'-(cyanométhyl)dihydrospectinomycine.

8. Composé de formule $II_b$

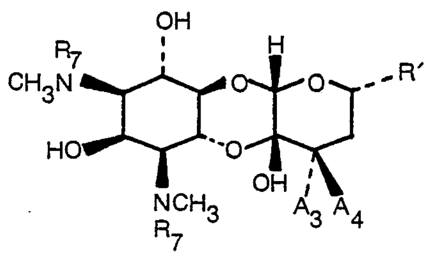

$II_b$

dans laquelle $R_7$ représente un groupe de blocage;

R' représente H, un groupe alkyle en $C_1$ à $C_8$, hydroxyalkyle en $C_1$ à $C_4$, (alcoxy en $C_1$ à $C_6$)-alkyle en $C_1$ à $C_4$ ayant au total jusqu'à 7 atomes de carbone, aminoalkyle en $C_1$ à $C_4$ bloqué sur N, (alkylamino en $C_1$ à $C_6$)-alkyle en $C_1$ à $C_4$ bloqué sur N, [di(alkyle en $C_1$ à $C_4$)amino]-alkyle en $C_1$ à $C_4$ dans lequel le groupe alkyle en $C_1$ à $C_4$ et chaque groupe alkyle en $C_1$ à $C_6$ possèdent conjointement jusqu'à 7 atomes de carbone, halogénalkyle en $C_1$ à $C_4$, dihalogénalkyle en $C_1$ à $C_4$ ou trihalogénalkyle en $C_1$ à $C_4$; et

$A_3$ et $A_4$ répondent aux définitions mentionnées pour $A_1$ et $A_2$ dans la revendication 1, ou bien l'un des groupes $A_3$ et $A_4$ représente un groupe OH et l'autre un groupe $CH_2N_3$.

9. Composé choisi entre
la N,N'-dibenzyloxycarbonyl-3'-(R)-3'-aminométhyldihydrospectinomycine;
la N,N'-dibenzyloxycarbonyl-3'-(S)-3'-aminométhyldihydrospectinomycine;
la N,N'-dibenzyloxycarbonyl-3'-(R)-3'-(N''-éthylaminométhyl)dihydrospectinomycine;
la N,N'-dibenzyloxycarbonyl-3'-(S)-3'-(N''-éthylaminométhyl)dihydrospectinomycine;
la N,N'-dibenzyloxycarbonyl-3'-(R)-3'-(N''-propylaminométhyl)dihydrospectinomycine;
la N,N'-dibenzyloxycarbonyl-3'-(R)-3'-(N''-butylaminométhyl)dihydrospectinomycine;
la N,N'-dibenzyloxycarbonyl-3'-(S)-3'-(N''-butylaminométhyl)dihydrospectinomycine;
la N,N'-dibenzyloxycarbonyl-3'-(R)-3'-(N''-isobutylaminométhyl)dihydrospectinomycine;

la N,N'-dibenzyloxycarbonyl-3'-(R)-3'-(N''-pentylaminométhyl)dihydrospectinomycine;
la N,N'-dibenzyloxycarbonyl-3'-(R)-3'-[N''-(2-méthylbutyl)-aminométhyl]dihydrospectinomycine;
la N,N'-dibenzyloxycarbonyl-3'-(R)-3'-(N''-hexylaminométhyl)dihydrospectinomycine;
la N,N'-dibenzyloxycarbonyl-3'-(R)-3'-(N''-octylaminométhyl)dihydrospectinomycine;
la N,N'-dibenzyloxycarbonyl-3'-(S)-3'-(N''-octylaminométhyl)dihydrospectinomycine;
la N,N'-dibenzyloxycarbonyl-3'-(R)-3'-(N'',N''-diosobutylaminométhyl)dihydrospectinomycine; et
la N,N'-dibenzyloxycarbonyl-3'-(N'',N''-dihexylaminométhyl)dihydrospectinomycine.

10. Composé choisi entre
la N,N'-dibenzyloxycarbonyl-3'-(R)-3'-(N''-cyclopentylaminométhyl)dihydrospectinomycine;
la N,N'-dibenzyloxycarbonyl-3'-(R)-3'-(N''-cyclohexylaminométhyl)dihydrospectinomycine;
la N,N'-dibenzyloxycarbonyl-3'-(R)-3'-(N''-cycloheptylaminométhyl)dihydrospectinomycine;
la N,N'-dibenzyloxycarbonyl-3'-(R)-3'-[N''-(cyclohexylméthyl)aminométhyl]dihydrospectinomycine;
la N,N'-dibenzyloxycarbonyl-3'-(R)-3'-[N''-(cyclooctylméthyl)aminométhyl]dihydrospectinomycine; et
la N,N'-dibenzyloxycarbonyl-3'-(R)-3'-[N'',N''-di(cyclohexylméthyl)aminométhyl]-dihydrospectinomycine.

11. Composé choisi entre
la N,N'-dibenzyloxycarbonyl-3'-(R)-3'-[N''-benzyloxycarbonylméthyl)aminométhyl]-dihydrospectinomycine;
la N,N'-dibenzyloxycarbonyl-3'-(R)-3'-(N''-acétylaminométhyl)dihydrospectinomycine; et
la N,N'-dibenzyloxycarbonyl-3'-(R)-3'-(N''-succinylaminométhyl)dihydrospectinomycine.

12. Composé choisi entre
la N,N'-dibenzyloxycarbonyl-3'-(azidométhyl)dihydrospectinomycine;
la N,N'-dibenzyloxycarbonyl-3'-(cyanométhyl)dihydrospectinomycine; et
la N,N'-dibenzyloxycarbonyl-3'-(R)spectinomycine-cyanhydrine.

13. Procédé de préparation d'un mélange d'épimères d'un composé suivant la revendication 1 dans lequel $A_1$ ou $A_2$ représente un groupe CN ou bien d'un mélange d'épimères d'un composé suivant la revendication 8 dans lequel $A_3$ ou $A_4$ représente un groupe CN, qui consiste à soumettre à une formation de cyanhydrine un composé de formule $III_{(b)}$

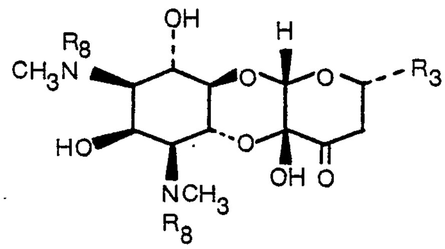

$III_{(b)}$

dans laquelle $R_8$ représente H et $R_3$ représente le groupe R tel qu'il a été défini dans la revendication 1 ou bien $R_8$ représente un groupe de blocage et $R_3$ représente le groupe R' tel qu'il a été défini dans la revendication 8, en présence d'une base ou d'une résine basique échangeuse d'ions, dans un solvant organique inerte; et à laisser le produit de réaction en solution pendant un temps suffisant pour que, de manière prédominante dans le mélange, $A_1$ ou $A_3$ représente un groupe CN; ou bien à terminer la réaction lorsque, de manière prédominante dans le mélange, $A_2$ ou $A_4$ représente un groupe CN, par addition d'un acide pour neutraliser la base ou la résine basique échangeuse d'ions ou bien, si aucune autre base n'est présente, par élimination de la résine.